# EUROPÄISCHE PATENTANMELDUNG

(11) **EP 4 806 426 A1**
(43) Veröffentlichungstag der Anmeldung: **16.09.2026**
(21) Anmeldenummer: 25162983.8
(22) Anmeldetag: 11.03.2025
(51) Int. Cl.: A61K 9/107, A61K 31/05, A61K 36/9066, A61K 47/02, A61K 47/24, A61K 47/36, A61K 47/46

(54) **VERFAHREN ZUR MIKROEMULGIERUNG LIPOPHILER SUBSTANZEN MITTELS EINER KOMBINATION AUS GUMMI ARABICUM, CHITOSAN, LECITHIN UND ALOE VERA**

(71) Anmelder: CannSol Science AG, 9491 Rugell (LI)
(72) Erfinder: PIDRONI, Angelo, 9487 Gamprin-Bendern (LI); GOOP, Franz Josef, 9488 Schellenberg (LI)
(74) Vertreter: Arth, Hans-Lothar

(57) **Zusammenfassung**

Die vorliegende Erfindung betrifft ein Verfahren zur Herstellung von Mikroemulsionen, das auf einer synergistischen Kombination aus Gummi arabicum (GA), Chitosan, Lecithin und Aloe vera als primäre Emulgatoren basiert. Ziel des Verfahrens ist die signifikante Steigerung der Resorptionseffizienz und Bioverfügbarkeit lipophiler Pflanzenwirkstoffe, Pflanzenextrakte, Vitamine und Vitalstoffe. Das Verfahren umfasst die Vorbehandlung der zu emulgierenden Substanzen, die Herstellung einer Trägerlösung und den kontrollierten Solubilisierungsprozess. Die resultierenden Emulsionen weisen stabile hydrodynamische Partikelgrößen zwischen 800 nm und 1200nm bzw. 200 nm und 800nm auf und eignen sich für Anwendungen in der Lebensmittel-, Kosmetik- und Nahrungsergänzungsmittelindustrie.

## Beschreibung

Die vorliegende Erfindung betrifft ein Verfahren zur Herstellung von Mikroemulsionen, das auf einer synergistischen Kombination aus Gummi arabicum (GA), Chitosan, Lecithin und Aloe vera als primäre Emulgatoren basiert. Ziel des Verfahrens ist die signifikante Steigerung der Resorptionseffizienz und Bioverfügbarkeit lipophiler Pflanzenwirkstoffe, Pflanzenextrakte, Vitamine und Vitalstoffe. Das Verfahren umfasst die Vorbehandlung der zu emulgierenden Substanzen, die Herstellung einer Trägerlösung und den kontrollierten Solubilisierungsprozess. Die resultierenden Emulsionen weisen stabile hydrodynamische Partikelgrößen zwischen 200 nm und 1200 nm, insbesondere zwischen 200 nm und 800 nm auf und eignen sich für Anwendungen in der Lebensmittel-, Kosmetik- und Nahrungsergänzungsmittelindustrie.

### Hintergrund der Erfindung

Lipophile Pflanzenwirkstoffe, Vitamine und Vitalstoffe oder auch essenzielle Fettsäuren aus Pflanzenölen spielen für die menschliche Ernährung und Gesundheit eine wesentliche Rolle. Ihre geringe Wasserlöslichkeit stellt jedoch eine zentrale Herausforderung bei der Entwicklung von hochbioverfügbaren Nahrungsergänzungsmitteln und Lebensmittelzusätzen dar. Aufgrund der eingeschränkten Löslichkeit werden sie im Verdauungstrakt in der Regel nur sehr begrenzt aufgenommen, was ihre Bioverfügbarkeit und Wirksamkeit erheblich reduziert. Um dennoch therapeutische oder funktionelle Effekte zu erzielen, sind oft hohe Dosen erforderlich, was das Risiko für potenzielle Nebenwirkungen signifikant erhöht. Abhängig vom Wirkstoff können hohe Tagesdosen zu Unverträglichkeiten führen, da das metabolische System des Körpers bei langfristiger Einnahme stark belastet und in seiner Funktion gestört werden kann. Ein erhöhtes Risiko besteht vor allem für empfindliche Personen oder bei der gleichzeitigen Einnahme von anderen Substanzen.

Konventionelle Ansätze zur Steigerung der Bioverfügbarkeit lipophiler Wirkstoffe umfassen unter anderem die Verwendung von Bioenhancern wie Piperin oder synthetischen Emulgatoren wie Polysorbaten. Piperin, ein natürlich vorkommendes Alkaloid aus schwarzem Pfeffer, wird häufig verwendet, um die Resorption von Wirkstoffen wie Curcumin zu verbessern. Die Wirkung von Piperin beruht auf der Hemmung bestimmter Phase-I- und Phase-II-Enzyme, wie beispielsweise Cytochrom-P450-Enzyme und UDP-Glucuronosyltransferasen. Dadurch wird der metabolische Abbau lipophiler Substanzen verlangsamt, was ihre systemische Verfügbarkeit erhöht. Dieser Ansatz ist jedoch mit mehreren Limitationen verbunden: Die Wirksamkeit von Piperin ist stark von individuellen Stoffwechselfaktoren abhängig und kann zudem die Resorption unerwünschter Substanzen oder Xenobiotika fördern. Hinzu kommt, dass eine langfristige Einnahme von Piperin die physiologischen Funktionen der Leber beeinträchtigt und pharmakokinetische Wechselwirkungen mit Medikamenten oder anderen Substanzen hervorrufen kann.

Polysorbate (z. B. Polysorbat 80 oder Polysorbat 20, auch bekannt als Tween 80 bzw. Tween 20) werden häufig als Emulgatoren in Lebensmitteln, Kosmetika und Pharmazeutika eingesetzt und gelten allgemein als sicher (GRAS-Status - Generally Recognized as Safe). Studien deuten jedoch darauf hin, dass Polysorbate die natürliche Balance der Darmmikrobiota stören können. Dies kann entzündungsfördernde Effekte hervorrufen und möglicherweise das Risiko für chronisch-entzündliche Darmerkrankungen erhöhen. Bei empfindlichen Personen oder bei höheren Dosierungen treten zudem häufig gastrointestinale Beschwerden wie Blähungen, Bauchschmerzen oder Durchfall auf. Nicht zuletzt können Polysorbate allergische Reaktionen hervorrufen und die Durchlässigkeit der Darmschleimhaut ("Leaky Gut") erhöhen, was systemische Entzündungsprozesse begünstigen kann. Ein weiterer Kritikpunkt ist ihre synthetische Herkunft, die nicht den wachsenden Anforderungen an natürliche und biokompatible Inhaltsstoffe entspricht, insbesondere in der Lebensmittel- und Nahrungsergänzungsmittelindustrie. Diese Nachteile schränken die Einsatzmöglichkeiten von Polysorbaten zunehmend ein und unterstreichen die Notwendigkeit, natürliche und effektivere Alternativen zu entwickeln.

Aufgabe der vorliegenden Erfindung ist die Bereitstellung eines Verfahrens zur Herstellung von stabilen Mikroemulsionen, die für eine gesteigerte Resorptionseffizienz und Bioverfügbarkeit lipophiler Pflanzenwirkstoffe, Pflanzenextrakte, Vitamine und Vitalstoffe bereitzustellen.

Diese Aufgabe wird erfindungsgemäß durch die technische Lehre der unabhängigen Ansprüche gelöst. Weitere vorteilhafte Ausgestaltungen der Erfindung ergeben sich aus den abhängigen Ansprüchen, der Beschreibung, den Figuren sowie den Beispielen.

### Kurzzusammenfassung

Die vorliegende Erfindung betrifft ein Verfahren zur Herstellung einer Mikroemulsion eines lipophilen Stoffes oder lipophilen Stoffgemisches umfassend die Schritte:
A) Bereitstellen einer Trägerlösung umfassend oder bestehend aus einer wässrigen Lösung eines physiologisch verträglichen Elektrolytsalzes und Lecithin mit einem Phosphatidylcholingehalt von mindestens 30 %;
B) Bereitstellen eines Pulverkonzentrats umfassend oder bestehend aus Gummi arabicum mit einem Gehalt an Arabinogalactanprotein von mindestens 3 Gew.-%, Chitosan mit einem Deacetylierungsgrad von mindestens 70%, Aloe vera-Gel/Pulver mit einem Gehalt an Acemannan von mindestens 10 Gew.-%; und dem lipophilen Stoff oder dem lipophilen Stoffgemisch;
C) Zufügen von maximal 30% (Gew./Vol.) des Pulverkonzentrats zur Trägerlösung zum Herstellen einer Pulverkonzentrat-Trägerlösung;
D) Homogenisieren der Pulverkonzentrat-Trägerlösung unter Bildung einer Mikroemulsion, wobei ein Einstellen des pH-Werts auf 4,0 erfolgt.

Die Technologie der Mikroemulgierung ermöglicht, die Art und Weise, wie lipophile Substanzen im Körper aufgenommen werden, grundlegend zu verbessern. Durch die Bildung solcher Emulsionen, in denen die Wirkstoffe in mizellenartigen Strukturen eingeschlossen sind, wird deren Löslichkeit und somit die Absorption im Verdauungstrakt erheblich verbessert.

In dem Verfahren der vorliegenden Erfindung wird Gummi arabicum (GA) verwendet. Im Vergleich zu den in der Pharmaindustrie häufig verwendeten Polysorbaten bietet GA spezifische Vorteile, die es zu einem bevorzugten Emulgator für die Herstellung von Nano- und Mikroemulsionen machen. Während Polysorbate äußerst effektiv in der Stabilisierung von Emulsionen sind, können sie jedoch Allergien auslösen und sind nicht für alle Patientengruppen geeignet. GA ist hingegen als "Generally Recognized As Safe" (GRAS) eingestuft und zeichnet sich durch eine hohe biologische Verträglichkeit aus. Zudem weist GA entzündungshemmende und antioxidative Eigenschaften auf, die zusätzliche therapeutische Vorteile bieten können. Die Verwendung von GA ermöglicht die Herstellung von mizellenartigen Strukturen, die eine effektive Solubilisierung und eine signifikant verbesserte Bioverfügbarkeit sicherstellen, ohne die Gesundheit des Endverbrauchers zu gefährden.

Die Herstellung stabilisierter Mikroemulsionen in wässrigen Systemen, speziell bei Konzentrationen nahe oder unterhalb der kritischen Mizellenkonzentration (CMC), erfordert eine präzise formulierte Trägerlösung. Diese Lösung dient nicht nur als Medium zur Bildung und Stabilisierung mizellenartiger Strukturen, sondern beeinflusst durch die gezielte Steuerung der Ionenstärke, des pH-Werts und der Grenzflächeninteraktionen entscheidend deren Stabilität und Funktionalität. Die Kombination aus GA, Chitosan, Lecitihin und Aloe vera spielt dabei eine synergistische Rolle, da sie die Bildung stabiler Grenzflächen fördert und eine langanhaltende physikalische Integrität der Hydrokolloide gewährleistet.

In bevorzugten Ausführungsformen umfasst das Verfahren weiter den Schritt E):
E) Behandeln der Mikroemulsion mit Ultraschall unter Bildung einer Mikroemulsion enthaltend Hydrokolloide mit einem durchschnittlichen hydrodynamischen Durchmesser zwischen 200 nm und 800 nm.

In bevorzugten Ausführungsformen wird Schritt C) bei einer Temperatur von ≤ 15°C durchgeführt.

In bevorzugten Ausführungsformen enthält die Trägerlösung 2 - 3 % (Gew./Vol.) Lecithin.

In bevorzugten Ausführungsformen wird das physiologisch verträgliche Elektrolytsalz ausgewählt wird aus der Gruppe umfassend oder bestehend aus Calciumchlorid, Natriumchlorid, Magnesiumchlorid, und Kaliumchlorid, bevorzugt Calciumchlorid. In bevorzugten Ausführungsformen enthält die Trägerlösung das physiologisch verträgliche Elektrolytsalz in einer Konzentration von ca. 9 bis 27 mM.

In bevorzugten Ausführungsformen weist das Chitosan einen Deacetylierungsgrad von mindestens 85% auf. In bevorzugten Ausführungsformen weist das Aloe vera-Gel/Pulver einen Gehalt an Acemannan von mindestens 15 Gew.-% auf.

In bevorzugten Ausführungsformen enthält die Mikroemulsion 1 - 5% (Gew./Vol.) lipophilen Stoff oder lipophiles Stoffgemisch, 10 - 25% (Gew./Vol.) Gummi arabicum, 0,5 - 1% (Gew./Vol.) Chitosan, 1 - 5% (Gew./Vol.) Aloe Vera-Gel/Pulver, 2 - 3% (Gew./Vol.) Lecithin und 0,1 - 0,3% (Gew./Vol.) Calciumchlorid.

In bevorzugten Ausführungsformen wird die Trägerlösung hergestellt durch ein Verfahren umfassend die Schritte:
a) Bereitstellen von Wasser, eines Salzes, bevorzugt Calciumchlorid, und Lecithin mit einem Phosphatidylcholingehalt von mindestens 30 %;
b) Temperieren des Wassers auf eine Temperatur zwischen 30°C - 45°C, bevorzugt 35°C;
c) Zufügen von 2 - 3 % (Gew./Vol.) des Lecithins;
d) Zufügen von 0,1 - 0,3 % (Gew./Vol.) des Salzes, bevorzugt Calciumchlorid;
e) Entgasen der Trägerlösung.

In bevorzugten Ausführungsformen wird das Pulverkonzentrat hergestellt durch ein Verfahren umfassend die Schritte:
i) Bereitstellen von Lösungsmittel, Gummi arabicum mit einem Gehalt an Arabinogalactanprotein von mindestens 3 Gew.-%, Chitosan mit einem Deacetylierungsgrad von mindestens 75%, Aloe vera-Gel/Pulver mit einem Gehalt an Acemannan von mindestens 10 Gew.-%; und lipophilen Stoff oder lipophilen Stoffgemisch;
ii) Lösen oder Dispergieren von 2 - 10 % (Gew./Vol.) des lipophilen Stoffs oder lipophilen Stoffgemischs im Lösungsmittel und Homogeniseren;
iii) Zufügen von 20 - 50 % (Gew./Vol.) des Gummi arabicums;
iv) Zufügen von 1 - 5 % (Gew./Vol.) des Chitosans;
v) Zufügen von 2 - 10 % (Gew./Vol.) des Aloe vera Gels/Pulvers;
vi) Homogenisieren;
vii) optionales Entgasen; und
viii) Entfernen des Lösungsmittels.

Bevorzugt ist das Lösungsmittel lebensmitteltaugliches Ethanol.

Bevorzugt wird der lipophile Stoff oder das lipophile Stoffgemisch ausgewählt aus der Gruppe umfassend oder besteht aus Hanfextrakte, Cannabinoide (Isolate), Curcuma-Extrakte, Curcuminoide (Isolate), Weihrauch-Extrakte, Boswelliasäuren (Isolate), Coenzym Q10, Ingwer-Extrakte, Ashwagandha-Extrakte, Oligomere Proanthocyanidine, Resveratrol, Vitamin A, D, E, K, Omega-3 Fettsäuren aus Fischöl oder Algenöl, Schwarzkümmelöl, Schwarzkümmelextrakte, Artemisia-Extrakte, Extrakte oder Ätherische Öle aus Oregano, Curcuma, Weihrauch, Ingwer, Lavendel, Thymian, Zitrone, Orange, Pfefferminze, Rosmarin, Eukalyptus, Basilikum, Schwarzkümmel, Nelke, Ylang-Ylang, Anis, Kardamon, Salbei, Zitronengras, Wachholderbeeren, Bergamotte, Zitronenverbene, Johanniskraut, Astaxanthin, Spermidin, Quercetin, Fisetin und NAD+.

Die vorliegende Erfindung betrifft zudem eine Mikroemulsion erhalten oder erhältlich nach einem Verfahren gemäß der vorliegenden Erfindung.

### Detaillierte Beschreibung

Überraschend wurde gefunden, dass stabile Mikroemulsionen von lipophilen Stoffen bzw. lipophilen Stoffgemischen durch ein Verfahren umfassend die folgenden Schritte hergestellt werden können:
A) Bereitstellen einer Trägerlösung umfassend oder bestehend aus einer wässrigen Lösung eines physiologisch verträglichen Elektrolytsalzes und Lecithin mit einem Phosphatidylcholingehalt von mindestens 30 %;
B) Bereitstellen eines Pulverkonzentrats umfassend oder bestehend aus Gummi arabicum mit einem Gehalt an Arabinogalactanprotein von mindestens 3 Gew.-%, Chitosan mit einem Deacetylierungsgrad von mindestens 70%, Aloe vera-Gel/Pulver mit einem Gehalt an Acemannan von mindestens 10 Gew.-%; und dem lipophilen Stoff oder dem lipophilen Stoffgemisch;
C) Zufügen von maximal 30% (Gew./Vol.) des Pulverkonzentrats zur Trägerlösung zum Herstellen einer Pulverkonzentrat-Trägerlösung;
D) Homogenisieren der Pulverkonzentrat-Trägerlösung unter Bildung einer Mikroemulsion, wobei ein Einstellen des pH-Werts auf 4,0 erfolgt.

Die resultierenden Mikroemulsionen bewirken eine signifikante Steigerung der Resorptionseffizienz und Bioverfügbarkeit lipophiler Pflanzenwirkstoffe, Pflanzenextrakte, Vitamine und Vitalstoffe. Die resultierenden Emulsionen weisen besonders bevorzugt stabile hydrodynamische Partikelgrößen zwischen 200 nm und 800 nm auf und eignen sich für Anwendungen in der Lebensmittel-, Kosmetik- und Nahrungsergänzungsmittelindustrie.

### Definitionen

Der Begriff "**Gummi arabicum**" (GA) (auch "Gummiarabikum", "Arabisches Gummi"), wie hierin verwendet, bezieht sich auf die gummiartigen Substanz (natürliches Harz) von in Afrika beheimateten Akazienarten, wie z.B. dem Gummiarabikumbaum (*Acacia senegal; Senegalia senegal*) oder der Seyal-Akazie (*Acacia seyal; Vachellia seyal*), die aus den Akazienbäumen durch Einschneiden des Stammes oder der Äste gewonnen wird. Die resultierenden Harzausscheidungen werden getrocknet und weiterverarbeitet. Gummi arabicum ist ein natürliches Gemisch von Polysacchariden mit dem Hauptbestandteil Arabinogalactan, das aus Arabinose- und Galactose-Monosacchariden besteht, wobei das Arabinogalactan teilweise an Proteine in Form von Arabinogalactanproteinen (AGPs) gebunden vorliegt. Da es sich bei Gummi arabicum um ein Naturprodukt handelt, unterliegt seine Zusammensetzung natürlichen Schwankungen. Gummi arabicum ist als Lebensmittelzusatzstoff E414 (CAS 9000-01-05) in Lebensmitteln als Verdickungsmittel, Emulgator und Stabilisator zugelassen und ist in den verschiedenen Reinheitsgraden und in Form von Flocken, Granulat, Pulver oder sprühgetrocknet erhältlich. Der genaue Gehalt an AGPs kann je nach Herkunft des Gummis variieren, wobei Gummi Arabicum aus *Acacia senegal* (*Senegalia senegal*) tendenziell mehr AGPs als das von *Acacia seyal* (*Vachellia seyal*) enthält. Im Schnitt enthält Gummi Arabicum aus *Acacia senegal* (*Senegalia senegal*) etwa 2-3 % Arabinogalactanproteine (AGPs) (bezogen auf das Gesamtgewicht). Das Gummi Arabicum aus *Acacia senegal* (*Senegalia senegal*) kann einen Gehalt von AGPs bis zu etwa 10 % des Gesamtgewichts aufweisen.

Gummi arabicum bildet die Grundlage des hierin beschriebenen Emulsionssystems. Überraschenderweise wurde gefunden, dass Gummi arabicum mit einem hohen Gehalt an Arabinogalactanproteinen (AGPs) von mindestens 3 Gew.-% eine zentrale Rolle bei der Stabilisierung der Mikroemulsionen der vorliegenden Erfindung spielt. Diese amphiphilen Moleküle besitzen sowohl hydrophile als auch hydrophobe Domänen, was sie zu effektiven Emulgatoren macht und ihre Funktion als Stabilisator von Emulsionssystemen unterstützt. Mit anderen Worten wurde überraschenderweise gefunden, dass für die Herstellung von hochstabilen Mikroemulsionen umfassend Hydrokolloide mit stabilen hydrodynamischen Partikelgrößen zwischen 200 und 1200 nm, insbesondere 200 nm und 800 nm, der Einsatz von Gummi arabicum mit einem Gehalt an AGPs von mindestens 3% erforderlich ist. Verfahren zur Bestimmung des Gehalts an Arabinogalactanprotein von Gummi arabicum sind aus dem Stand der Technik bekannt, z.B. lässt sich der Gehalt an AGPs mittels ELISA-Test (Enzyme-Linked Immunosorbent Assay) oder Kapillarelektrophorese (CE) bestimmen (Referenzmethode: ISO 19657 ("Spezifikation für Gummi arabicum")). Beim ELISA erfolgt die Quantifizierung durch Antikörper-Antigen-Reaktion und bei der Kapillarelektrophorese (CE) erfolgt eine Auftrennung der Polysaccharid-Protein-Komplexe nach Größe und Ladung. Alternativ kann auch die Phenol-Schwefelsäure-Methode zur allgemeinen Polysaccharid-Bestimmung (unspezifisch) verwendet werden. Für die Fachperson auf dem Gebiet stellt die Bestimmung des Gehalts an AGPs in Gummi arabicum somit eine routinemäßige Aufgabe dar.

In bevorzugten Ausführungsformen wird Gummi arabicum mit einem Gehalt an AGPs von mindestens 3% vom Typ *Acacia senegal* oder *Acacia seyal* verwendet. In weiter bevorzugten Ausführungsformen wird Gummi arabicum mit einem Gehalt an AGPs von mindestens 3% vom Typ *Acacia senegal* verwendet. In bevorzugten Ausführungsformen wird sprühgetrocknetes Gummi arabicum mit einem Gehalt an AGPs von mindestens 3% verwendet. In weiter bevorzugten Ausführungsformen wird sprühgetrocknetes Gummi arabicum mit einem Gehalt an AGPs von mindestens 3% vom Typ *Acacia senegal* oder *Acacia seyal* verwendet. In besonders bevorzugten Ausführungsformen wird sprühgetrocknetes Gummi arabicum mit einem Gehalt an AGPs von mindestens 3% vom Typ *Acacia senegal* verwendet.

Erfindungsgemäß weist das Gummi arabicum einen Gehalt an Arabinogalactanprotein von mindestens 3 Gew.-% auf. Weiterhin kann Gummi arabicum auch mit einem Gehalt an Arabinogalactanprotein von mindestens 4 Gew.-%, mindestens 5 Gew.-%, mindestens 6 Gew.-%, mindestens 7 Gew.-%, weiter mindestens 8 Gew.-%, mindestens 9 Gew.-%, oder auch mindestens 10 Gew.-% zur Herstellung der Mikroemulsion der vorliegenden Erfindung verwendet werden.

Der Begriff "**Chitosan**" ("Poliglusam", "Poly-D-Glucosamin", "Polyglucosamin"), wie hierin verwendet, bezieht sich auf ein natürlich vorkommendes Biopolymer, das durch Deacetylierung von Chitin gewonnen wird - einem Hauptbestandteil der Exoskelette von Krustentieren (z. B. Krabben, Garnelen) sowie der Zellwände von Pilzen. Dabei wird Chitin durch Entfernen der Acetylgruppen zu Chitosan umgewandelt. Der Grad der resultierenden Deacetylierung kann erheblich variieren. Der genaue Grad der Deacetylierung (der Anteil an freien Aminogruppen) und das Molekulargewicht beeinflussen dabei die Löslichkeit und die funktionellen Eigenschaften von Chitosan. Die Deacetylierung kann vollständig oder teilweise erfolgen, woraus eine Verteilung stark deacetylierter neben wenig deacetylierten Bereichen oder eine homogene Deacetylierungsverteilung resultieren kann, was erhebliche Auswirkungen auf die Molekülgestalt hat. Gleichzeitig kann aufgrund dieses chemischen Eingriffes die Kettenlänge des Polymers abnehmen (Depolymerisation), wodurch die Löslichkeit verbessert und die Viskosität verringert wird. Die Endprodukte können sich daher erheblich in ihren Eigenschaften unterscheiden. Der Deacetylierungsgrad von Chitosan variiert je nach Quelle und Herstellungsprozess und liegt typischerweise bei mindestens 60 % bis zu etwa 100%.

Überraschenderweise wurde gefunden, dass Chitosan mit einem hohen Deacetylierungsgrad von mindestens 70 % eine zentrale Rolle bei der Stabilisierung der Mikroemulsionen der vorliegenden Erfindung spielt, da die kationischen Eigenschaften des Chitosans die Wechselwirkung mit den anionischen AGPs aus Gummi Arabicum verbessert. Durch die Interaktion von AGPs mit Chitosan entsteht ein stabilisierendes Konjugat. Aufgrund seiner kationischen Ladung kann Chitosan mit den anionischen Carboxylgruppen der AGPs elektrostatische Interaktionen eingehen, die zur Bildung eines stabilisierenden Komplexes führen. Verfahren zur Bestimmung des Deacetylierungsgrads von Chitosan sind aus dem Stand der Technik bekannt, z.B. Fourier-Transform-Infrarotspektroskopie (FTIR) oder Kernspinresonanzspektroskopie (¹H-NMR) (Referenzmethode: ISO 17226 ("Bestimmung des Deacetylierungsgrads in Chitosan")). Dabei wird bei der FTIR das Verhältnis der Absorptionsbanden von Amiden und Hydroxylgruppen und bei der ¹H-NMR-Analyse über die Integration der Peaks für N-Acetyl-Glucosamin- und Glucosamin-Einheiten der Deacetylierungsgrad bestimmt. Alternativ kann auch eine Leitfähigkeits-Titration nach Hydrolyse des Chitosans erfolgen. Für die Fachperson auf dem Gebiet stellt die Bestimmung des Deacetylierungsgrads von Chitosan somit eine routinemäßige Aufgabe dar.

Erfindungsgemäß weist das Chitosan einen Deacetylierungsgrad von mindestens 70 % auf. In besonders bevorzugten Ausführungsformen weist das Chitosan einen Deacetylierungsgrad von mindestens 85 % auf. Chitosan kann aber auch einen Deacetylierungsgrad von bis zu 95 % aufweisen. Somit weist das Chitosan erfindungsgemäß einen Deacetylierungsgrad von 70 % - 95 % auf. In einigen Ausführungsformen weist Chitosan einen Deacetylierungsgrad von 70 % - 75 % auf. Weiterhin kann in einigen bevorzugten Ausführungsformen das Chitosan einen Deacetylierungsgrad von 90 % - 95 % aufweisen.

In bevorzugten Ausführungsformen weist Chitosan einen Deacetylierungsgrad von mindestens 71 %, weiter bevorzugt mindestens 72%, weiter bevorzugt mindestens 73%, weiter bevorzugt mindestens 74%, weiter bevorzugt mindestens 75%, weiter bevorzugt mindestens 76%, weiter bevorzugt mindestens 77%, weiter bevorzugt mindestens 78%, weiter bevorzugt mindestens 79%, weiter bevorzugt mindestens 80%, weiter bevorzugt mindestens 81%, weiter bevorzugt mindestens 82%, weiter bevorzugt mindestens 83%, weiter bevorzugt mindestens 84%, besonders bevorzugt mindestens 85% auf.

In besonders bevorzugten Ausführungsformen weist das Chitosan einen Deacetylierungsgrad von 80 % - 90 %, weiter bevorzugt von 81 % - 89 %, weiter bevorzugt von 82 % - 88 %, weiter bevorzugt von 83 % - 87 %, und am meisten bevorzugt von 84 % - 86 % auf. Insbesondere bevorzugt ist ein Deacetylierungsgrad von 85 %.

Einige Pilze enthalten neben Chitin auch Chitosan in ihrer Zellwand, so dass das Chitosan aus diesen direkt gewonnen werden kann. Alle bekannten Arten wie z. B. *Mucor rouxii*, *Absidia coerulea* und *Rhizopus oryzae* gehören zur Ordnung der Mucorales. Somit kann Chitosan aus den Zellwänden bestimmter Pilzarten (z. B. *Mucor spp.)* direkt gewonnen. Der Deacetylierungsgrad von Chitosan aus *Mucor spp., R. oryzae* und *H. illucens* liegt im Bereich von mindestens 80%. Chitosan, das aus Pilzen gewonnen wird hat in der Regel einen sehr hohen Deacetylierungsgrad, oft etwa 90 %. Dies liegt daran, dass die enzymatische Deacetylierung bei Pilzen häufig kontrollierter verläuft als die chemische Deacetylierung von Chitin aus Krebstieren, wo der Grad variieren kann.

In bevorzugten Ausführungsformen wird daher hochwertiges Chitosan fungalen Ursprungs verwendet, das aus den Zellwänden bestimmter Pilzarten (z. B. *Mucor* spp. oder *Rhizopus spp*.) gewonnen wird. Das verwendete Chitosan fungalen Ursprungs zeichnet sich insbesondere durch einen gleichmäßigen hohen Grad der Deacetylierung (mindestens 80 %) aus.

Der Begriff "**Aloe vera**", wie hierin verwendet, bezieht sich auf eine Pflanze aus der Gattung der Aloen, d.h. auf die "Echte Aloe", die mit fast 400 weiteren Pflanzenarten zu der Gattung der Aloen (Aloe) gehört. Der wissenschaftliche Name der Echten Aloe (Aloe vera) lautet Aloe vera barbadensis MILLER. Die Pflanze ist insbesondere bekannt für ihre dicken, fleischigen Blätter, die ein klares Gel enthalten. Die Blätter der Aloe vera bestehen aus zwei Hauptkomponenten, der äußeren grünen Rinde und dem inneren farblosen Parenchym (Aloe-vera-Filet), welches das Gel der Aloe vera enthält. Das Blattinnere der Aloe vera besteht zu 98,5% aus Wasser (99,5 % bezogen auf Aloe-vera-Gel) und enthält über 75 beschriebene Inhaltsstoffe, darunter Polysaccharide, Vitamine, organische Säuren und Glucose. Aloverose (Acemannan, Glucomannan), ein aus β-(1→4)-verknüpften D-Mannoseeinheiten aufgebautes acetyliertes Polysaccharid, wird als wertgebender Bestandteil des Aloe-vera-Gels angesehen. Der Begriff "**Aloe vera-Gel**", wie hierin verwendet, bezieht sich auf das Gel aus dem Blattinneren der Aloe vera Pflanze. Der Begriff "**Aloe vera-Pulver**", wie hierin verwendet, bezieht sich auf getrocknetes Aloe vera-Gel. Um Aloe Vera Pulver herzustellen, wird das Aloe vera-Gel zunächst dehydriert, wodurch das Wasser entzogen wird. Das resultierende Pulver enthält eine konzentrierte Form der aktiven Bestandteile der Pflanze. Die Qualität des Pulvers wird oft durch den Gehalt an Polysacchariden wie Acemannan bestimmt, die für die gesundheitlichen Vorteile von Aloe Vera verantwortlich sind. Während Aloe Vera Gel aufgrund seines hohen Wassergehalts eine begrenzte Haltbarkeit hat und kühl gelagert werden sollte, ist das Pulver durch die Dehydration länger haltbar und leichter zu transportieren. Das Pulver kann bei Bedarf rehydriert oder direkt in verschiedenen Formulierungen verwendet werden, was es vielseitig einsetzbar macht.

Wie hierin verwendet bezieht sich der Begriff Aloe vera-Gel sowohl auf das Gel das direkt aus dem Inneren der Blätter der Aloe Vera Pflanze gewonnen wird und aus zu etwa 99,5 % aus Wasser besteht, als auch auf das getrocknetes Aloe vera-Gel, d.h. das Aloe vera-Pulver, bei dem das Wasser entzogen wurde. Frisches Aloe-Vera-Gel enthält natürliche Mengen an Acemannan, die jedoch variieren können. Der Vorteil von Aloe vera-Pulver besteht darin, dass Aloe vera-Pulver eine konzentrierte Form des Acemannans bietet, wobei der genaue Gehalt von der Qualität des Produkts abhängt. Besonders bevorzugt ist daher die Verwendung von Aloe vera-Pulver für die Herstellung der Mikroemulsionen der vorliegenden Erfindung, allerdings ist die Verwendung des wasserhaltigen frischen Aloe vera-Gels nicht ausgeschlossen.

Das Aloe vera-Gel, insbesondere das Aloe vera-Pulver, ergänzt das Mikroemulsionssystem der vorliegenden Erfindung durch eine Vielzahl bioaktiver Inhaltsstoffe, darunter insbesondere Polysaccharide wie Acemannan, Mannose und Glukuronsäure. Diese Substanzen stabilisieren vorteilhaft die Emulsion, indem sie ein hydrophiles Netzwerk bilden, das die mikrostrukturelle Homogenität der dispergierten Partikel aufrechterhält und deren Koaleszenz weiter verhindert. Gleichzeitig tragen diese Polysaccharide zur Stabilisierung der Viskosität bei, was insbesondere für die Herstellung langzeitstabiler Emulsionen von Vorteil ist. Aloe vera-Pulver enthält zudem kleine Mengen an Saponinen, welche die Oberflächenspannung reduzieren und dadurch die Emulgierungseffizienz des Systems weiter erhöhen können. Die Emulsion erreicht dadurch ein Zeta-Potenzial von ca. -40 mV, was eine hohe elektrostatische Abstoßung zwischen den Partikeln gewährleistet.

Überraschenderweise wurde gefunden, dass Aloe vera-Gel in Form von Aloe vera-Pulver mit einem hohen Gehalt an Acemannan von mindestens 10 Gew.-% eine zentrale Rolle bei der Stabilisierung der Mikroemulsionen der vorliegenden Erfindung spielt. Verfahren zur Bestimmung des Acemannan-Gehalts in Aloe-vera sind aus dem Stand der Technik bekannt, z. B. Hochleistungsflüssigchromatographie (HPLC) oder Fourier-Transform-Infrarotspektroskopie (FTIR) (Referenzmethode: ISO 19619:2018 ("Bestimmung von Acemannan in Aloe-vera-Rohstoffen und Endprodukten")). Dabei erfolgt bei der HPLC eine Analyse des Acemannan-Polysaccharids durch Retentionszeit und Peak-Integration, und bei der FTIR eine Identifizierung der charakteristischen Polysaccharid-Banden. Alternativ kann auch die Carbazol-Schwefelsäure-Methode zur Bestimmung von Polysacchariden verwendet werden. Für die Fachperson auf dem Gebiet stellt die Bestimmung des Gehalts an Acemannan in Aloe vera-Gel somit eine routinemäßige Aufgabe dar. Eine gängige Methode zur Bestimmung von Aloverose, einem Indikator für Acemannan, ist die Anwendung spezifischer Farbreaktionen. Diese Methode ermöglicht es, den

Gehalt an Aloverose im Aloe-vera-Gel zu bestimmen, der typischerweise zwischen 5 und 20 % der Trockenmasse beträgt.

Erfindungsgemäß weist das Aloe vera-Pulver einen Gehalt an Acemannan von mindestens 10 Gew.-% auf. In besonders bevorzugten Ausführungsformen weist das Aloe vera-Pulver einen Gehalt an Acemannan von mindestens 15 Gew.-% auf. Aloe vera-Pulver kann aber auch einen Gehalt an Acemannan von bis zu 20 Gew.- % aufweisen. Somit weist das Aloe vera-Pulver einen Gehalt an Acemannan von 10 Gew.-% - 20 Gew.-% auf. In einigen Ausführungsformen das Aloe vera-Pulver einen Gehalt an Acemannan von 10 Gew.-% - 15 Gew.-% auf. Weiterhin kann in einigen bevorzugten Ausführungsformen das Aloe vera-Pulver einen Gehalt an Acemannan von 15 Gew.-% - 20 Gew.-% aufweisen. In einigen Ausführungsformen weist das Aloe vera-Pulver einen Gehalt an Acemannan von 10 Gew.-% - 20 Gew.-%, weiter bevorzugt von 11 Gew.-% - 19 Gew.-%, weiter bevorzugt von 12 Gew.-% - 18 Gew.-%, weiter bevorzugt von 13 Gew.-% - 17 Gew.-%, besonders bevorzugt von 14 Gew.-% - 16 Gew.-%.

In einigen bevorzugten Ausführungsformen weist Aloe vera-Pulver einen Gehalt an Acemannan von mindestens 11 Gew.-%, weiter bevorzugt mindestens 12 Gew.-%, weiter bevorzugt mindestens 13 Gew.-%, weiter bevorzugt mindestens 14 Gew.-%, und insbesondere bevorzugt mindestens 15 Gew.-%.

In einigen bevorzugten Ausführungsformen weist Aloe vera-Pulver einen Gehalt an Acemannan von 11 Gew.-%, weiter bevorzugt 12 Gew.-%, weiter bevorzugt 13 Gew.-%, weiter bevorzugt 14 Gew.-%, und insbesondere bevorzugt 15 Gew.-%.

Im Fall der Verwendung von wasserhaltigen frischem Aloe vera-Gel beziehen sich die Angaben bezüglich des Acemannan-Gehalts hierin auf die Trockenmasse. Mit anderen Worten weist das Aloe vera-Gel einen Gehalt an Acemannan von mindestens 10 Gew.-% bezogen auf die Trockenmasse auf. In besonders bevorzugten Ausführungsformen weist das Aloe vera-Gel einen Gehalt an Acemannan von mindestens 15 Gew.-% bezogen auf die Trockenmasse auf. Aloe vera-Gel kann aber auch einen Gehalt an Acemannan von bis zu 20 Gew.- % bezogen auf die Trockenmasse aufweisen. Somit weist das Aloe vera Gel einen Gehalt an Acemannan von 10 Gew.-% - 20 Gew.-% bezogen auf die Trockenmasse auf. In einigen Ausführungsformen weist das Aloe vera-Gel einen Gehalt an Acemannan von 10 Gew.-% - 15 Gew.-% bezogen auf die Trockenmasse auf. Weiterhin kann in einigen bevorzugten Ausführungsformen das Aloe vera-Gel einen Gehalt an Acemannan von 15 Gew.-% - 20 Gew.-% bezogen auf die Trockenmasse aufweisen. In einigen Ausführungsformen weist das Aloe vera-Gel einen Gehalt an Acemannan von 10 Gew.-% - 20 Gew.-% bezogen auf die Trockenmasse, weiter bevorzugt von 11 Gew.-% - 19 Gew.-% bezogen auf die Trockenmasse, weiter bevorzugt von 12 Gew.-% - 18 Gew.-% bezogen auf die Trockenmasse, weiter bevorzugt von 13 Gew.-% - 17 Gew.-% bezogen auf die Trockenmasse, besonders bevorzugt von 14 Gew.-% - 16 Gew.-% bezogen auf die Trockenmasse auf.

In einigen bevorzugten Ausführungsformen weist Aloe vera-Gel einen Gehalt an Acemannan von mindestens 11 Gew.-% bezogen auf die Trockenmasse, weiter bevorzugt mindestens 12 Gew.-% bezogen auf die Trockenmasse, weiter bevorzugt mindestens 13 Gew.-% bezogen auf die Trockenmasse, weiter bevorzugt mindestens 14 Gew.-% bezogen auf die Trockenmasse, und insbesondere bevorzugt mindestens 15 Gew.-% bezogen auf die Trockenmasse auf.

In einigen bevorzugten Ausführungsformen weist Aloe vera-Gel einen Gehalt an Acemannan von 11 Gew.-% bezogen auf die Trockenmasse, weiter bevorzugt 12 Gew.-% bezogen auf die Trockenmasse, weiter bevorzugt 13 Gew.-% bezogen auf die Trockenmasse, weiter bevorzugt 14 Gew.-% bezogen auf die Trockenmasse, und insbesondere bevorzugt 15 Gew.-% bezogen auf die Trockenmasse auf.

Der Begriff "**Lecithin**", wie hierin verwendet, bezieht sich auf einen Bestandteil tierischer und pflanzlicher Zellmembranen und gehört zur Gruppe der Phospholipide und wird auch als "Phosphatidylcholin" bezeichnet. Dabei handelt es sich um Phospholipide, die Ester aus Fettsäuren, Glycerin, Phosphorsäure und Cholin darstellen. Lecithine erlauben das Emulgieren von Fetten und Wasser und sind somit wichtige natürliche Emulgatoren für Lebens- und Futtermittel. In der EU sind sie als Lebensmittelzusatzstoff (E 322) für Lebensmittel zugelassen. In der Medizin und in der Kosmetik werden sie auch als Wirkstoff, in der Ernährung zu diätetischen Zwecken und zur Nahrungsergänzung genutzt.

Überraschenderweise wurde gefunden, dass Lecithin einen Phosphatidylcholingehalt von mindestens 30 Gew.-% eine zentrale Rolle bei der Stabilisierung der Mikroemulsionen der vorliegenden Erfindung spielt. Verfahren zur Bestimmung des Phosphatidylcholingehalts in Lecithin sind aus dem Stand der Technik bekannt, z. B. Hochleistungsflüssigchromatographie (HPLC) mit Evaporative Light Scattering Detection (ELSD) (Referenzmethode: ISO 10540-1 ("Bestimmung von Phosphatidylcholin in Lecithin")). Dabei erfolgt die Trennung der Phospholipid-Fraktionen und spezifische Quantifizierung des Phosphatidylcholins. Alternativ kann auch Dünnschichtchromatographie (TLC) in Kombination mit Densitometrie verwendet werden. Für die Fachperson auf dem Gebiet stellt die Bestimmung des Gehalts an Phosphatidylcholin in Lecithin somit eine routinemäßige Aufgabe dar.

Besonders bevorzugt ist hochreines, fettfreies, entgummiertes Lecithin pflanzlichen Ursprungs (z. B. aus Sonnenblumen oder Sojabohnen). Das Lecithin sollte möglichst vollständig fettfrei sein und einen Phosphatidylcholingehalt von mindestens 30 % und bevorzugt mindestens 45 % aufweisen. Diese Eigenschaften sind entscheidend, da Phosphatidylcholin ein wesentlicher Emulgator ist, der die Mizellisierung von lipophilen Pflanzenextrakten fördern kann.

Erfindungsgemäß weist das Lecithin einen Phosphatidylcholingehalt von mindestens 30 Gew.-% auf. In bevorzugten Ausführungsformen weist das Lecithin einen Phosphatidylcholingehalt von mindestens 40 Gew.-% auf. In weiter bevorzugten Ausführungsformen weist das Lecithin einen Phosphatidylcholingehalt von mindestens 45 Gew.-% auf. Lecithin kann auch einen Phosphatidylcholingehalt von bis zu 75 Gew.-% aufweisen. Somit weist das Lecithin erfindungsgemäß einen Phosphatidylcholingehalt von 30 Gew.-% - 75 Gew.-% auf. In einigen Ausführungsformen weist das Lecithin einen Phosphatidylcholingehalt bevorzugt von 35 Gew.-% - 45 Gew.-% auf. Weiterhin kann in einigen bevorzugten Ausführungsformen das Lecithin einen Phosphatidylcholingehalt von 40 Gew.-% - 50 Gew.-% aufweisen.

In einigen bevorzugten Ausführungsformen weist das Lecithin einen Phosphatidylcholingehalt von 35 Gew.-% - 60 Gew.-%, weiter bevorzugt von 40 Gew.-% - 50 Gew.-%, weiter bevorzugt von 41 Gew.-% - 49 Gew.-%, weiter bevorzugt von 42 Gew.-% - 48 Gew.-%, weiter bevorzugt von 43 Gew.-% - 47 Gew.-%, besonders bevorzugt von 44 Gew.-% - 46 Gew.-% auf.

In bevorzugten Ausführungsformen weist Lecithin einen Phosphatidylcholingehalt von mindestens 31 Gew.-%, weiter bevorzugt mindestens 32 Gew.-%, weiter bevorzugt mindestens 33 Gew.-%, weiter bevorzugt mindestens 34 Gew.-%, weiter bevorzugt mindestens 35 Gew.-%, weiter bevorzugt mindestens 36 Gew.-%, weiter bevorzugt mindestens 37 Gew.-%, weiter bevorzugt mindestens 38 Gew.-%, weiter bevorzugt mindestens 39 Gew.-%, weiter bevorzugt mindestens 40 Gew.-%, weiter bevorzugt mindestens 41 Gew.-%, weiter bevorzugt mindestens 42 Gew.-%, weiter bevorzugt mindestens 43 Gew.-%, weiter bevorzugt mindestens 44 Gew.-%, besonders bevorzugt mindestens 45 Gew.-% auf.

In bevorzugten Ausführungsformen weist Lecithin einen Phosphatidylcholingehalt von 31 Gew.-%, weiter bevorzugt von 32 Gew.-%, weiter bevorzugt von 33 Gew.-%, weiter bevorzugt von 34 Gew.-%, weiter bevorzugt von 35 Gew.-%, weiter bevorzugt von 36 Gew.-%, weiter bevorzugt von 37 Gew.-%, weiter bevorzugt von 38 Gew.-%, weiter bevorzugt von 39 Gew.-%, weiter bevorzugt von 40 Gew.-%, weiter bevorzugt von 41 Gew.-%, weiter bevorzugt von 42 Gew.-%, weiter bevorzugt von 43 Gew.-%, weiter bevorzugt von 44 Gew.-%, besonders bevorzugt von 45 Gew.-% auf.

Der Begriff "**physiologisch verträgliches Elektrolytsalz",** wie hierin verwendet, bezeichnet ionische Verbindungen, die in wässriger Lösung in positiv und negativ geladene Ionen (Kationen und Anionen) dissoziieren und somit den elektrischen Strom leiten können. Sie spielen eine entscheidende Rolle in vielen biologischen Prozessen, wie der Regulierung des Wasserhaushalts, der Nervenleitung und der Muskelkontraktion. "Physiologisch verträglich", wie hierin verwendet, bedeutet das ein Stoff oder eine Substanz vom Körper gut aufgenommen und verarbeitet wird, ohne negative Auswirkungen auf die Gesundheit zu haben. Mit anderen Worten bezeichnet "physiologisch verträglich" die Eigenschaft einer Substanz, die normalen Lebensvorgänge im Körper nicht zu beeinträchtigen und somit als sicher und unbedenklich für den menschlichen Organismus zu gelten. Ein "physiologisch verträgliches Elektrolytsalz" ist somit eine ionische Verbindung, die in wässriger Lösung in positiv und negativ geladene Ionen (Kationen und Anionen) dissoziiert und sicher und unbedenklich für den menschlichen Organismus ist.

"Physiologisch verträgliche Elektrolytsalze" bieten die Fähigkeit, die Ionenstärke der Trägerlösung gezielt zu erhöhen, ohne die Struktur der Hydrokolloid-basierten Mizellen zu destabilisieren. Alkalimetall- und Erdalkalimetall-Salze, insbesondere von Chloriden, zeichnen sich durch eine vollständige Dissoziation, eine hohe Stabilität und eine gute Kompatibilität mit den verwendeten Emulgatoren aus. Bevorzugte physiologisch verträgliche Elektrolytsalze schließen daher ein, sind aber nicht beschränkt auf Calciumchlorid, Natriumchlorid, Magnesiumchlorid, und Kaliumchlorid, besonders bevorzugt ist Calciumchlorid.

Die Anforderungen an ein physiologisch verträgliches Elektrolytsalz sind folgende:
1. Hohe Wasserlöslichkeit:
   Vollständige Dissoziation in Ionen zur effektiven Steigerung der Ionenstärke.
   Löslichkeit ≥ 1 g/100 ml bei 20°C.
2. Neutraler oder schwach saurer Charakter:
   Kein Einfluss auf den pH-Wert der Trägerlösung.
   Keine/geringe Reaktion mit Gummi Arabicum (GA), Aloe vera-Gel/Pulver (AV), Chitosan oder Lecithin.
3. Kompatibilität mit Hydrokolloiden:
   Keine Fällungsreaktionen oder Koagulation mit den verwendeten Emulgatoren.
   Erhalt der Hydrokolloidstruktur.
   Keine Zersetzung oder Bildung unerwünschter Nebenprodukte.
   Förderung der Stabilität der Mikroemulsion.
   Effiziente Erhöhung der elektrolytischen Leitfähigkeit.
4. Physiologische Verträglichkeit:
   Lebensmitteltauglich, pharmazeutisch oder kosmetisch unbedenklich.
   Vorzugsweise Alkali- oder Erdalkalimetall-Salze.

Insbesondere hat sich gezeigt, dass Calciumionen (Ca²⁺) besonders vorteilhaft sind, um die Ionenstärke der Lösung zu steigern, da diese die elektrostatischen Wechselwirkungen innerhalb des Systems gezielt beeinflussen. Dies verbessert insbesondere die Bindung zwischen anionischen Gruppen (z. B. Carboxylgruppen von Polysacchariden wie Acemannan aus Aloe vera oder AGPs aus Gummi arabicum) und kationischen Molekülen (z. B. Chitosan). Eine erhöhte Ionenstärke kann die Vernetzung und Stabilisierung der Emulsion fördern, indem sie die elektrostatischen Bindungen an den Grenzflächen stärkt. Dies verbessert die mechanische Festigkeit der Grenzflächenschicht und erhöht die physikalische Integrität der Tropfenstruktur. Zu hohe Ionenstärken können jedoch destabilisieren, indem sie die elektrostatische Abschirmung zu stark erhöhen. In besonders bevorzugten Ausführungsformen ist das physiologisch verträgliche Elektrolytsalz Calciumchlorid.

Der Begriff "**Lösungsmittel**", wie hierin verwendet, bezieht sich auf einen Stoff, hierin eine Flüssigkeit, der Gase, andere Flüssigkeiten oder Feststoffe lösen kann, ohne dass es dabei zu chemischen Reaktionen zwischen gelöstem Stoff und lösendem Stoff kommt. Für die vorliegende Erfindung werden insbesondere lebensmitteltaugliche Lösungsmittel verwendet. Lebensmitteltaugliche Lösungsmittel sind beispielsweise Wasser oder Ethanol. Für die Herstellung des Pulverkonzentrats wird erfindungsgemäß ein lebensmitteltaugliches Lösungsmittel verwendet, welches im letzten Schritt möglichst vollständig entfernt wird, um ein getrocknetes Pulver zu erhalten. Hierbei hat sich insbesondere die Verwendung von lebensmitteltauglichem Ethanol als besonders geeignet erwiesen. Daher wird zur Herstellung des Pulverkonzentrats gemäß der vorliegenden Erfindung besonders bevorzugt lebensmitteltaugliches Ethanol verwendet. Lebensmitteltaugliches Ethanol wird häufig als Lösungsmittel in der Lebensmittelindustrie eingesetzt, beispielsweise zur Extraktion von Aromen oder Wirkstoffen. Es gibt jedoch Alternativen, die je nach Anwendung in Betracht gezogen werden können. Beispielsweise ist die Verwendung von Glycerin als Lösungsmittel und natürliches Konservierungsmittel aus dem Stand der Technik bekannt. Obwohl es weniger effektiv als Ethanol ist und möglicherweise größere Mengen an Rohstoffen für die Extraktion benötigt werden, wird es oft in Kombination mit Wasser verwendet, insbesondere wenn auf Ethanol verzichtet werden soll. Gemäß der Verordnung (EU) 2020/771 dürfen zur Extraktion in der Lebensmittelindustrie auch Lösungsmittel wie Aceton, Methanol, Hexan, Propan-2-ol, Ethylacetat, alkalischer Alkohol oder überkritisches Kohlendioxid verwendet werden.

Die Begriffe "**gereinigtes Wasser**", "**Reinstwasser**", "**vollentsalztes Wasser**" (VE-Wasser) und "**destilliertes Wasser**", wie hierin verwendet, beziehen sich auf unterschiedliche Reinheitsgrade und Herstellungsverfahren von Wasser. Bekannte Herstellungsverfahren sind Destillation, kapazitive Deionisierung, Umkehrosmose, Kohlefilterung, Mikrofiltration, Ultrafiltration, Ultraviolettoxidation oder Elektrodeionisation und Kombinationen davon. Der Begriff "**gereinigtes Wasser**", wie hierin verwendet, bezieht sich auf Wasser, das mechanisch gefiltert oder verarbeitet wurde, um Verunreinigungen zu entfernen und es für den Gebrauch geeignet zu machen. Gereinigtes Wasser wird aus Trinkwasser durch Verfahren wie Ionenaustausch, Umkehrosmose oder Destillation hergestellt. Es enthält noch einen gewissen Restgehalt an bestimmten Ionen und weist eine spezifische Leitfähigkeit bei 25 °C von 1-50 µS/cm auf. Der Begriff "**Reinstwasser**", wie hierin verwendet, betrifft Wasser mit einem sehr hohen Reinheitsgrad und das durch mehrstufige Verfahren wie Umkehrosmose, Ionenaustausch, Filtration und UV-Bestrahlung hergestellt wurde. Reinstwasser weist eine äußerst geringe elektrische Leitfähigkeit auf. Der theoretische Grenzwert liegt bei 0,055 µS/cm (Mikrosiemens pro Zentimeter) bei 25 °C. Der Begriff "**vollentsalztes Wasser"** (VE-Wasser) oder "demineralisiertes Wasser" oder "deionisiertes Wasser", betrifft Wasser, dem die im normalen Quell- und Leitungswasser vorkommenden gelösten Salze weitgehend entzogen wurden. Dies wird durch Ionenaustausch oder Umkehrosmose erreicht. VE-Wasser enthält jedoch möglicherweise noch organische Stoffe oder Mikroorganismen, da diese Verfahren hauptsächlich Ionen entfernen. Der Begriff "**destilliertes Wasser**", wie hierin verwendet, betrifft Wasser, das durch Destillation gewonnen wird, wobei Wasser verdampft und anschließend kondensiert wird. Dieser Prozess entfernt nicht nur Salze und Mineralien, sondern auch viele organische Verbindungen und Mikroorganismen, was zu einer höheren Reinheit führt. Die elektrische Leitfähigkeit von destilliertem Wasser liegt typischerweise zwischen 0,5 und 5 µS/cm bei 25 °C.

In bevorzugten Ausführungsformen wird für die Herstellung der Mikroemulsion der vorliegenden Erfindung mindestens gereinigtes Wasser oder vollentsalztes Wasser (VE-Wasser) verwendet. Weiter bevorzugt wird destilliertes Wasser oder Reinstwasser verwendet. Am meistens bevorzugt wird Reinstwasser verwendet.

Der Begriff "**lebensmitteltaugliche Säure**", wie hierin verwendet, bezieht sich auf organische oder anorganische Säuren, die als Lebensmittelzusatzstoffe zugelassen sind und in der Lebensmittelindustrie verwendet werden, um den Geschmack, die Haltbarkeit oder die Textur von Produkten zu beeinflussen. Mit lebensmitteltauglichen Säuren ist es möglich, den pH-Wert der Mikroemulsionen der vorliegenden Erfindung auf einen gewünschten pH-Wert einzustellen. Zur Einstellung des pH-Werts in Lebensmitteln oder auch Nahrungsergänzungsmitteln werden im Stand der Technik verschiedene lebensmitteltaugliche Säuren verwendet, die sowohl die Stabilität als auch die Wirksamkeit der Produkte beeinflussen können. Da insbesondere ein pH-Wert von 4,0 der resultierenden Mikroemulsion gewünscht ist, sind vor allem schwache organische Säuren oder Lebensmittel- bzw. pharmazeutisch zugelassene Säuren geeignet. Diese müssen mit den verwendeten Emulgatoren verträglich sein. Empfohlene Säuren schließen daher ein, sind aber nicht beschränkt auf Zitronensäure (Citronensäure, E330), die bevorzugt ist, da stabil, geschmacksneutral und pufferfähig und zusammen mit ihrem Salz (Natriumcitrat) eine stabile Pufferwirkung bietet, Ascorbinsäure (Vitamin C, E300), die bevorzugt ist, da sie wirksam ist und zusätzlich antioxidative Eigenschaften aufweist und den Oxidationsschutz der Mikroemulsion verbessern kann, Milchsäure (L(+)-Milchsäure, E270), die sehr gut wasserlöslich, mild wirksam, pH-stabilisierend ist, und Essigsäure (E260), die effektiv, aber aufgrund ihres Geruchs eher nachrangig, aber dennoch nicht ausgeschlossen ist. Andere bekannte lebensmitteltaugliche Säuren sind Weinsäure (E 334), die natürlich in vielen Früchten vorkommt und als Säuerungsmittel in verschiedenen Lebensmitteln und Nahrungsergänzungsmitteln verwendet wird und Äpfelsäure (E 296), die sich in vielen Früchten findet und als Säuerungsmittel in Lebensmitteln verwendet wird.

Nicht empfohlene Säuren sind beispielsweise starke Säuren wie Salzsäure oder Schwefelsäure, da sie zur Destabilisierung/Denaturierung der Hydrokolloide in der Mikroemulsion führen können.

Grundsätzlich kann der pH-Wert während oder nach der Homogenisierung eingestellt werden. Vor der Homogenisierung ist nicht empfohlen, aber nicht grundsätzlich ausgeschlossen.

Der Begriff "**lipophiler Stoff**", wie hierin verwendet, bezieht sich im Allgemeinen auf eine Substanz, die sich gut in Fetten, Ölen und unpolaren Lösungsmitteln löst. Hierin bezieht sich der Begriff lipophiler Stoff auf lipophile Stoffe, die in der Natur vorkommen, z. B. die aus Pflanzen gewonnen werden und beispielsweise in Nahrungsergänzungsmitteln Verwendung finden. Einige Beispiele von lipophilen Stoffen schließen ein, sind aber nicht beschränkt auf Fettsäuren, z.B. essentielle Fettsäuren wie Omega-3- und Omega-6-Fettsäuren, die aus pflanzlichen Ölen wie Leinöl oder Hanföl gewonnen werden können, Carotinoide, die als fettlösliche Pigmente, wie Beta-Carotin oder Lutein, in Karotten, Spinat und anderen grünen Blattgemüsen vorkommen, Phytosterole, die pflanzliche Sterole sind, die in Ölen wie Soja- oder Rapsöl vorkommen und zur Senkung des Cholesterinspiegels beitragen können, Vitamin E, das ein fettlösliches Vitamin ist, das in Pflanzenölen, Nüssen und Samen vorkommt und antioxidative Eigenschaften besitzt. Der Begriff "**lipophiles Stoffgemisch**", wie hierin verwendet, bezieht sich auf eine Mischung von lipophilen Stoffen, aber auch auf ein Naturprodukt, wie ein Pflanzenextrakt, bei dem neben dem gewünschten lipophilen Stoff noch weitere Stoffe enthalten sind.

Der Begriff "**Pflanzenextrakte**", wie hierin verwendet bezieht sich auf konzentrierte Substanzen, die durch das Herauslösen spezifischer Inhaltsstoffe aus Pflanzen gewonnen werden. Dieser Prozess, bekannt als Extraktion, nutzt Lösungsmittel wie Wasser, Alkohol oder Glycerin, um die gewünschten Wirkstoffe aus verschiedenen Pflanzenteilen wie Blättern, Blüten, Wurzeln, Samen oder Rinden zu isolieren. Die Zusammensetzung von Pflanzenextrakten variiert je nach verwendeter Pflanze und Extraktionsmethode. Typische Bestandteile sind Vitamine, Mineralstoffe, Flavonoide, Alkaloide, und Terpene. Diese Inhaltsstoffe verleihen Pflanzenextrakten ihre spezifischen Eigenschaften und Anwendungen, beispielsweise in der Kosmetik, Medizin oder als Nahrungsergänzungsmittel. Pflanzenextrakte stellen hierin somit Stoffgemische dar, da diese nicht aus einem reinen isolierten Stoff bestehen. "**Lipophile Pflanzenextrakte**" sind konzentrierte Präparate, die fettlösliche (lipophile) Inhaltsstoffe aus Pflanzen enthalten. Diese Extrakte werden durch spezifische Extraktionsverfahren gewonnen, bei denen Lösungsmittel wie Alkohol oder andere organische Substanzen verwendet werden, um die gewünschten fettlöslichen Komponenten aus den Pflanzen zu isolieren. Typische lipophile Inhaltsstoffe in Pflanzenextrakten sind Ätherische Öle, Fettsäuren, Carotinoide und Phytosterole. Lipophile Pflanzenextrakte werden in der Kosmetik, z. B. in Hautpflegeprodukten in der Lebensmittelindustrie, z.B. als natürliche Aromen, Farbstoffe oder Antioxidantien, und in der pharmazeutischen Industrie, z.B. als Basis für pflanzliche Arzneimittel und als Inhaltstoffe für Nahrungsergänzungsmittel verwendet.

Der Begriff "**Vitamin**", wie hierin verwendet, bezieht sich auf eine organische Verbindung, die ein Organismus nicht als Energieträger, sondern für andere lebenswichtige Funktionen benötigt, die jedoch der Stoffwechsel nicht bedarfsdeckend synthetisieren kann. Vitamine müssen mit der Nahrung aufgenommen werden, sie gehören zu den essentiellen Stoffen. Man unterteilt Vitamine in fettlösliche (lipophile) und wasserlösliche (hydrophile) Vitamine. Chemisch bilden die Vitamine keine einheitliche Stoffgruppe. "**Lipophile Vitamine**", auch als fettlösliche Vitamine bekannt, sind Vitamine, die sich in Fetten und Ölen lösen und im Körper gespeichert werden können. Zu dieser Gruppe gehören die Vitamine A, D, E und K.

Der Begriff "**Vitalstoff**", wie hierin verwendet, bezieht sich auf essentielle Nährstoffe, die der menschliche Körper benötigt, um optimal zu funktionieren. Sie umfassen Vitamine, Mineralstoffe, Spurenelemente und weitere bioaktive Substanzen. Diese Stoffe spielen eine entscheidende Rolle bei der Stärkung des Immunsystems, dem Erhalt gesunder Knochen und der Unterstützung des Stoffwechsels. **Lipophile Vitalstoffe** sind fettlösliche Nährstoffe, die sich in Fetten und Ölen lösen und im Körper gespeichert werden können. Zu dieser Gruppe gehören die Vitamine A, D, E und K. Weitere lipophile Vitalstoffe, die für den menschlichen Körper wichtig sind, schließen ein, sind aber nicht beschränkt auf Coenzym Q10 (Ubichinon), Carotinoide wie Beta-Carotin, Lutein und Lycopin, und Alkylglycerole.

Der Begriff "**Pflanzenwirkstoffe**", wie hierin verwendet, bezieht sich auf bioaktive Substanzen, die von Pflanzen produziert werden und vielfältige Funktionen erfüllen. Ein Großteil der Pflanzenwirkstoffe fällt unter die Kategorie der sekundären Pflanzenstoffe wie z.B. Flavonoide, Carotinoide, Glucosinolate, Phytosterole und Phytoöstrogene, welche nicht am primären Stoffwechsel der Pflanze beteiligt sind, aber jedoch wichtige ökologische Funktionen übernehmen. "**Lipophile Pflanzenstoffe**" sind fettlösliche Substanzen, die in Pflanzen vorkommen. Aufgrund ihrer Lipophilie lösen sie sich in Fetten und Ölen und nicht in Wasser. Diese Eigenschaft beeinflusst ihre Absorption und Funktion im menschlichen Körper.

Bevorzugt sind die "**lipophilen Stoffe**" bzw. "**lipophilen Stoffgemische**", die bei der Herstellung der Mikroemulsionen der vorliegenden Erfindung verwendet werden daher lipophile Pflanzenextrakte, lipophile Vitamine, lipophile Vitalstoffe bzw. lipophile Pflanzenstoffe.

Lipophile Stoffe oder lipophile Stoffgemische, die bei der Herstellung der Mikroemulsionen der vorliegenden Erfindung verwendet werden können, schließen ein, sind aber nicht beschränkt auf Hanfextrakte, Cannabinoide (Isolate), Curcuma-Extrakte, Curcuminoide (Isolate), Weihrauch-Extrakte, Boswelliasäuren (Isolate), Coenzym Q10, Ingwer-Extrakte, Ashwagandha-Extrakte, Oligomere Proanthocyanidine, Resveratrol, Vitamin A, Vitamin D, Vitamin E, Vitamin K, omega-3 Fettsäuren aus Fischöl oder Algenöl, Schwarzkümmelöl, Schwarzkümmelextrakte, Artemisia-Extrakte, Extrakte oder Ätherische Öle aus Oregano, Curcuma, Weihrauch, Ingwer, Lavendel, Thymian, Zitrone, Orange, Pfefferminze, Rosmarin, Eukalyptus, Basilikum, Schwarzkümmel, Nelke, Ylang-Ylang, Anis, Kardamon, Salbei, Zitronengras, Wachholderbeeren, Bergamotte, Zitronenverbene, Johanniskraut, Astaxanthin, Spermidin, Quercetin, Fisetin und NAD+.

Die vorgenannten aufgelisteten Substanzen und Extrakte sind lipophile (fettlösliche) Verbindungen, die in verschiedenen Bereichen wie Nahrungsergänzungsmitteln, der Medizin und der Kosmetik Anwendung finden. Hanfextrakte und Cannabinoide (Isolate) enthalten bioaktive Verbindungen wie Cannabidiol (CBD) und Tetrahydrocannabinol (THC), die für ihre entspannenden und entzündungshemmenden Eigenschaften bekannt sind, Curcuma-Extrakte und Curcuminoide (Isolate) beinhalten Curcumin als Hauptwirkstoff das starke antioxidative und entzündungshemmende Eigenschaften besitzt, Boswelliasäuren haben entzündungshemmende Wirkungen und werden traditionell zur Linderung von Gelenkbeschwerden eingesetzt, Ingwer-Extrakte enthalten Gingerole und Shogaole, die antioxidative und entzündungshemmende Effekte haben. Ashwagandha-Extrakte werden in der ayurvedischen Medizin verwendet, um Stress zu reduzieren und das allgemeine Wohlbefinden zu fördern, oligomere Proanthocyanidine (OPC) sind starke Antioxidantien, die in Traubenkernen und anderen Pflanzen vorkommen und Zellschäden durch freie Radikale verhindern können, Resveratrol ist ein Polyphenol, das in Trauben und Beeren vorkommt und antioxidative sowie entzündungshemmende Eigenschaften besitzt, die Vitamine A, Vitamin D, Vitamin E, Vitamin K sind fettlösliche Vitamine, die essentielle Funktionen im Körper erfüllen, wie z. B. Sehkraft (Vitamin A), Calciumstoffwechsel (Vitamin D), Schutz vor oxidativem Stress (Vitamin E) und Blutgerinnung (Vitamin K), omega-3-Fettsäuren aus Fischöl oder Algenöl sind essentielle Fettsäuren, die entzündungshemmende Eigenschaften haben und für die Herzgesundheit wichtig sind, Schwarzkümmelöl und Schwarzkümmelextrakte sind bekannt für ihre immunmodulierenden und entzündungshemmenden Effekte, Extrakte oder ätherische Öle aus Oregano, Curcuma, Weihrauch, Ingwer, Lavendel, Thymian, Zitrone, Orange, Pfefferminze, Rosmarin, Eukalyptus, Basilikum, Nelke, Ylang-Ylang, Anis, Kardamom, Salbei, Zitronengras, Wacholderbeeren, Bergamotte, Zitronenverbene und Johanniskraut: enthalten vielfältige lipophile Verbindungen, die in der Aromatherapie, als Duftstoffe oder wegen ihrer antimikrobiellen und entzündungshemmenden Eigenschaften genutzt werden, Coenzym Q10 ist ein lipophiles Molekül, das in den Mitochondrien der Zellen vorkommt und eine zentrale Rolle in der zellulären Energieproduktion spielt. Es wirkt zudem als Antioxidans und schützt Zellen vor oxidativem Stress. All diese lipophilen Substanzen zeichnen sich durch ihre Löslichkeit in Fetten und Ölen aus, was ihre Absorption im menschlichen Körper negativ beeinflusst. Sie werden häufig in Nahrungsergänzungsmitteln eingesetzt, um verschiedene gesundheitliche Vorteile zu bieten.

Neben den bereits genannten lipophilen Stoffen und lipophilen Stoffgemischen gibt es weitere Beispiele, wie Phytosterole bzw. pflanzliche Sterole, die strukturell dem Cholesterin ähneln und zur Senkung des Cholesterinspiegels im menschlichen Körper beitragen können, Squalen, ein natürlich vorkommendes Triterpen, das in pflanzlichen Ölen wie Olivenöl vorkommt und in der Kosmetik für seine feuchtigkeitsspendenden Eigenschaften genutzt wird, Lycopin, ein rotes Carotinoid, das hauptsächlich in Tomaten vorkommt und starke antioxidative Eigenschaften besitzt, Lutein, ein gelbes Carotinoid, das in grünem Blattgemüse wie Spinat und Grünkohl vorkommt und für die Augengesundheit wichtig ist, Zeaxanthin, ein Carotinoid, das in Mais, Paprika und Eigelb vorkommt und zusammen mit Lutein zur Gesundheit der Augen beiträgt, β-Carotin, ein Provitamin A, das in Karotten und anderen orangefarbenen Gemüsesorten vorkommt und im Körper zu Vitamin A umgewandelt wird, Tocopherole (Vitamin E), Antioxidantien, die in pflanzlichen Ölen, Nüssen und Samen vorkommen und Zellmembranen vor oxidativem Stress schützen, Phyllochinon (Vitamin K₁), das in grünem Blattgemüse vorkommt und wichtig für die Blutgerinnung ist, Menachinon (Vitamin K₂), das von Darmbakterien produziert wird und eine Rolle im Knochenstoffwechsel spielt, Chlorophyll, das grüne Pigment in Pflanzen, das für die Photosynthese verantwortlich ist und antioxidative Eigenschaften besitzt, Capsaicin, der scharfe Wirkstoff in Chilischoten, der schmerzlindernde Eigenschaften hat und in topischen Schmerzmitteln verwendet wird, Menthol, ein Monoterpen, das in Pfefferminze vorkommt und für seinen kühlenden Effekt bekannt ist, Thymol, ein Monoterpen, das in Thymian vorkommt und antimikrobielle Eigenschaften besitzt, Carvacrol, ein Monoterpen, das in Oregano vorkommt und starke antimikrobielle Eigenschaften aufweist, Glycyrrhizinsäure, ein Saponin aus der Süßholzwurzel mit entzündungshemmenden und antiviralen Eigenschaften, Genistein, ein Isoflavon aus Sojabohnen mit antioxidativen und östrogenähnlichen Eigenschaften, Daidzein, ein weiteres Isoflavon aus Soja mit ähnlichen Eigenschaften wie Genistein, Hesperidin, ein Flavonoid, das in Zitrusfrüchten vorkommt und antioxidative sowie entzündungshemmende Eigenschaften besitzt, Naringenin, ein Flavonoid aus Grapefruit mit antioxidativen und entzündungshemmenden Effekten, Quercetin, ein Flavonoid, das in Äpfeln, Zwiebeln und Beeren vorkommt und starke antioxidative Eigenschaften hat. Diese vorgenannten lipophilen Substanzen werden aufgrund ihrer vielfältigen biologischen Aktivitäten in der Medizin, Kosmetik und Lebensmittelindustrie geschätzt.

Die vorliegende Erfindung betrifft ein Verfahren zur Herstellung einer Mikroemulsion eines lipophilen Stoffes oder lipophilen Stoffgemisches, wobei einerseits eine Trägerlösung bereitgestellt wird, die als Lösungsmittel Wasser enthält, in dem ein physiologisch verträgliches Elektrolytsalz gelöst ist und wobei Lecithin in der Trägerlösung vorliegt, und wobei andererseits ein Pulverkonzentrat bereitgestellt wird, das sich aus Gummi Arabicum, Chitosan und Aloe vera-Gel/Pulver und dem gewünschten lipophilen Stoff oder lipophilen Stoffgemisches zusammensetzt. Durch kontrollierte Zugabe des Pulverkonzentrats zur Trägerlösung und anschließender Homogenisierung können so stabile Mikroemulsionen mit Hydrokolloiden gewünschter Größe erhalten werden, die vorteilhaften Eigenschaften gegenüber Mikroemulsionen aus dem Stand der Technik aufzeigen.

Es hat sich überraschenderweise gezeigt, dass das Zusammenspiel von Gummi Arabicum (GA), Chitosan, Lecithin und Aloe vera-Gel/Pulver die Herstellung hochstabiler Mikroemulsionen zur effektiven Solubilisierung lipophiler Wirkstoffe ermöglicht. Dieses vollständig natürliche und biokompatible System bietet eine innovative Alternative zu synthetischen Emulgatoren und erfüllt die wachsenden Anforderungen an sichere und nachhaltige Inhaltsstoffe in der Lebensmittel-, Kosmetik- und Nahrungsergänzungsmittelindustrie. Durch die Interaktion von Arabinogalactanproteinen (AGPs) mit Chitosan, einem Biopolymer, entsteht ein stabilisierendes Konjugat.

Das Verfahren zur Herstellung einer Mikroemulsion eines lipophilen Stoffes oder lipophilen Stoffgemisches umfasst daher die Schritte:
A) Bereitstellen einer Trägerlösung umfassend oder bestehend aus einer wässrigen Lösung eines physiologisch verträglichen Elektrolytsalzes und Lecithin mit einem Phosphatidylcholingehalt von mindestens 30%;
B) Bereitstellen eines Pulverkonzentrats umfassend oder bestehend aus Gummi arabicum mit einem Gehalt an Arabinogalactanprotein von mindestens 3 Gew.-%, Chitosan mit einem Deacetylierungsgrad von mindestens 70%, Aloe vera-Gel/Pulver mit einem Gehalt an Acemannan von mindestens 10 Gew.-%; und dem lipophilen Stoff oder dem lipophilen Stoffgemisch;
C) Zufügen von maximal 30% (Gew./Vol.) des Pulverkonzentrats zur Trägerlösung zum Herstellen einer Pulverkonzentrat-Trägerlösung;
D) Homogenisieren der Pulverkonzentrat-Trägerlösung unter Bildung einer Mikroemulsion, wobei ein Einstellen des pH-Werts auf 4,0 erfolgt.

Je nach lipophilen Stoffes oder lipophilen Stoffgemisches ergibt dieses Verfahren eine Mikroemulsion enthaltend Hydrokolloide mit einem durchschnittlichen hydrodynamischen Durchmesser zwischen 800 nm und 1.200 nm.

Das Verfahren zur Herstellung einer Mikroemulsion eines lipophilen Stoffes oder lipophilen Stoffgemisches umfasst daher bevorzugt die Schritte:
A) Bereitstellen einer Trägerlösung umfassend oder bestehend aus einer wässrigen Lösung eines physiologisch verträglichen Elektrolytsalzes und Lecithin mit einem Phosphatidylcholingehalt von mindestens 30%;
B) Bereitstellen eines Pulverkonzentrats umfassend oder bestehend aus Gummi arabicum mit einem Gehalt an Arabinogalactanprotein von mindestens 3 Gew.-%, Chitosan mit einem Deacetylierungsgrad von mindestens 70%, Aloe vera-Gel/Pulver mit einem Gehalt an Acemannan von mindestens 10 Gew.-%; und dem lipophilen Stoff oder dem lipophilen Stoffgemisch;
C) Zufügen von maximal 30% (Gew./Vol.) des Pulverkonzentrats zur Trägerlösung zum Herstellen einer Pulverkonzentrat-Trägerlösung;
D) Homogenisieren der Pulverkonzentrat-Trägerlösung unter Bildung einer Mikroemulsion enthaltend Hydrokolloide mit einem durchschnittlichen hydrodynamischen Durchmesser zwischen 800 nm und 1.200 nm, wobei ein Einstellen des pH-Werts auf 4,0 erfolgt.

Eine Sedimentationskontrolle kann durch Zentrifugation durchgeführt werden, um die physikalische Stabilität der Hydrokolloide zu überprüfen. Dazu werden z.B. 10 mL der Lösung für 60 Sekunden bei 2.500 × g zentrifugiert. Es sollte sich dabei kein Pellet bilden; ein Ausfallen der Partikel würde auf eine unzureichende Stabilisierung hinweisen.

Um eine kleinere Partikelgröße im Bereich zwischen 200 nm und 800 nm zu erreichen, kann optional anschließend eine Ultraschallbehandlung eingesetzt werden. Die optimale Ultraschallintensität und der erforderliche Energieeintrag können von der Fachperson in Vorversuchen bestimmt werden. Dazu wird nach verschiedenen Energieeinträgen die Partikelgröße mittels dynamischer Lichtstreuung (DLS) analysiert. Es ist wichtig zu beachten, dass eine zu hohe Ultraschallintensität die Hydrokolloidstrukturen zerstören kann.

In einigen bevorzugten Ausführungsformen umfasst das Verfahren daher bevorzugt weiter den Schritt E):
E) Behandeln der Mikroemulsion mit Ultraschall unter Bildung einer Mikroemulsion enthaltend Hydrokolloide mit einem durchschnittlichen hydrodynamischen Durchmesser zwischen 200 nm und 800 nm.

In einer bevorzugten Ausführungsform umfasst das Verfahren zur Herstellung einer Mikroemulsion eines lipophilen Stoffes oder lipophilen Stoffgemisches daher bevorzugt die Schritte:
A) Bereitstellen einer Trägerlösung umfassend oder bestehend aus einer wässrigen Lösung eines physiologisch verträglichen Elektrolytsalzes und Lecithin mit einem Phosphatidylcholingehalt von mindestens 30%;
B) Bereitstellen eines Pulverkonzentrats umfassend oder bestehend aus Gummi arabicum mit einem Gehalt an Arabinogalactanprotein von mindestens 3 Gew.-%, Chitosan mit einem Deacetylierungsgrad von mindestens 70%, Aloe vera-Gel/Pulver mit einem Gehalt an Acemannan von mindestens 10 Gew.-%; und dem lipophilen Stoff oder dem lipophilen Stoffgemisch;
C) Zufügen von maximal 30% (Gew./Vol.) des Pulverkonzentrats zur Trägerlösung zum Herstellen einer Pulverkonzentrat-Trägerlösung;
D) Homogenisieren der Pulverkonzentrat-Trägerlösung unter Bildung einer Mikroemulsion, wobei ein Einstellen des pH-Werts auf 4,0 erfolgt.
E) Behandeln der Mikroemulsion mit Ultraschall unter Bildung einer Mikroemulsion enthaltend Hydrokolloide mit einem durchschnittlichen hydrodynamischen Durchmesser zwischen 200 nm und 800 nm.

Mit anderen Worten umfasst das Verfahren zur Herstellung einer Mikroemulsion eines lipophilen Stoffes oder lipophilen Stoffgemisches daher bevorzugt die folgenden Schritte:
A) Bereitstellen einer Trägerlösung umfassend oder bestehend aus einer wässrigen Lösung eines physiologisch verträglichen Elektrolytsalzes und Lecithin mit einem Phosphatidylcholingehalt von mindestens 30%;
B) Bereitstellen eines Pulverkonzentrats umfassend oder bestehend aus Gummi arabicum mit einem Gehalt an Arabinogalactanprotein von mindestens 3 Gew.-%, Chitosan mit einem Deacetylierungsgrad von mindestens 70%, Aloe vera-Gel/Pulver mit einem Gehalt an Acemannan von mindestens 10 Gew.-%; und dem lipophilen Stoff oder dem lipophilen Stoffgemisch;
C) Zufügen von maximal 30% (Gew./Vol.) des Pulverkonzentrats zur Trägerlösung zum Herstellen einer Pulverkonzentrat-Trägerlösung;
D) Homogenisieren der Pulverkonzentrat-Trägerlösung unter Bildung einer Mikroemulsion enthaltend Hydrokolloide mit einem durchschnittlichen hydrodynamischen Durchmesser zwischen 800 nm und 1.200 nm, wobei ein Einstellen des pH-Werts auf 4,0 erfolgt.
E) Behandeln der Mikroemulsion mit Ultraschall unter Bildung einer Mikroemulsion enthaltend Hydrokolloide mit einem durchschnittlichen hydrodynamischen Durchmesser zwischen 200 nm und 800 nm.

Das physiologisch verträgliche Elektrolytsalz wird bevorzugt ausgewählt wird aus der Gruppe umfassend oder bestehend aus Calciumchlorid, Natriumchlorid, Magnesiumchlorid, und Kaliumchlorid, bevorzugt Calciumchlorid. **In** besonders bevorzugten Ausführungsformen ist das physiologisch verträgliche Elektrolytsalz Calciumchlorid. In bevorzugten Ausführungsformen enthält die Trägerlösung das physiologisch verträgliche Elektrolytsalz in einer Konzentration von ca. 9 bis 27 mM.

In bevorzugten Ausführungsformen wird das Wasser zur Herstellung der Mikroemulsion ausgewählt wird aus gereinigtem Wasser, vollentsalztem Wasser, destilliertem Wasser und Reinstwasser. Am meistens bevorzugt wird Reinstwasser als Lösungsmittel für die Trägerlösung bzw. die Mikroemulsion verwendet.

Bevorzugt ist daher ein Verfahren zur Herstellung einer Mikroemulsion eines lipophilen Stoffes oder lipophilen Stoffgemisches umfassend die Schritte:
A) Bereitstellen einer Trägerlösung umfassend oder bestehend aus einer wässrigen Lösung eines physiologisch verträglichen Elektrolytsalzes und Lecithin mit einem Phosphatidylcholingehalt von mindestens 30%;
   wobei das physiologisch verträgliche Elektrolytsalz ausgewählt wird aus der Gruppe umfassend oder bestehend aus Calciumchlorid, Natriumchlorid, Magnesiumchlorid, und Kaliumchlorid, bevorzugt Calciumchlorid; und/oder
   wobei gereinigtes Wasser, vollentsalztes Wasser, destilliertes Wasser oder Reinstwasser, bevorzugt Reinstwasser verwendet wird;
B) Bereitstellen eines Pulverkonzentrats umfassend oder bestehend aus Gummi arabicum mit einem Gehalt an Arabinogalactanprotein von mindestens 3 Gew.-%, Chitosan mit einem Deacetylierungsgrad von mindestens 70%, Aloe vera-Gel/Pulver mit einem Gehalt an Acemannan von mindestens 10 Gew.-%; und dem lipophilen Stoff oder dem lipophilen Stoffgemisch;
C) Zufügen von maximal 30% (Gew./Vol.) des Pulverkonzentrats zur Trägerlösung zum Herstellen einer Pulverkonzentrat-Trägerlösung;
D) Homogenisieren der Pulverkonzentrat-Trägerlösung unter Bildung einer Mikroemulsion enthaltend Hydrokolloide mit einem durchschnittlichen hydrodynamischen Durchmesser zwischen 800 nm und 1.200 nm, wobei ein Einstellen des pH-Werts auf 4,0 erfolgt.

Bevorzugt ist daher ein Verfahren zur Herstellung einer Mikroemulsion eines lipophilen Stoffes oder lipophilen Stoffgemisches umfassend die Schritte:
A) Bereitstellen einer Trägerlösung umfassend oder bestehend aus einer wässrigen Lösung von Reinstwasser, einem physiologisch verträglichen Elektrolytsalz und Lecithin mit einem Phosphatidylcholingehalt von mindestens 30 %;
   wobei das physiologisch verträgliche Elektrolytsalz ausgewählt wird aus der Gruppe umfassend oder bestehend aus Calciumchlorid, Natriumchlorid, Magnesiumchlorid, und Kaliumchlorid, bevorzugt Calciumchlorid;
B) Bereitstellen eines Pulverkonzentrats umfassend oder bestehend aus Gummi arabicum mit einem Gehalt an Arabinogalactanprotein von mindestens 3 Gew.-%, Chitosan mit einem Deacetylierungsgrad von mindestens 70%, Aloe vera-Gel/Pulver mit einem Gehalt an Acemannan von mindestens 10 Gew.-%; und dem lipophilen Stoff oder dem lipophilen Stoffgemisch;
C) Zufügen von maximal 30% (Gew./Vol.) des Pulverkonzentrats zur Trägerlösung zum Herstellen einer Pulverkonzentrat-Trägerlösung;
D) Homogenisieren der Pulverkonzentrat-Trägerlösung unter Bildung einer Mikroemulsion enthaltend Hydrokolloide mit einem durchschnittlichen hydrodynamischen Durchmesser zwischen 800 nm und 1.200 nm, wobei ein Einstellen des pH-Werts auf 4,0 erfolgt.

Das Einstellen des pH-Werts auf 4,0 erfolgt durch Zugabe von lebensmitteltauglichen Säuren, wie hierin definiert, d.h. bevorzugt mit Zitronensäure (E330), Ascorbinsäure (E300), Milchsäure (E270), oder Essigsäure (E260), bevorzugt Zitronensäure oder Ascorbinsäure, besonders bevorzugt Zitronensäure. Der pH-Wert wird bevorzugt während oder nach der Homogenisierung eingestellt, wobei das Einstellen des pH-Werts vor der Homogenisierung zwar nicht grundsätzlich ausgeschlossen, aber nicht empfohlen ist.

Bevorzugt ist daher ein Verfahren zur Herstellung einer Mikroemulsion eines lipophilen Stoffes oder lipophilen Stoffgemisches umfassend die Schritte:
A) Bereitstellen einer Trägerlösung umfassend oder bestehend aus einer wässrigen Lösung eines physiologisch verträglichen Elektrolytsalzes und Lecithin mit einem Phosphatidylcholingehalt von mindestens 30%;
B) Bereitstellen eines Pulverkonzentrats umfassend oder bestehend aus Gummi arabicum mit einem Gehalt an Arabinogalactanprotein von mindestens 3 Gew.-%, Chitosan mit einem Deacetylierungsgrad von mindestens 70%, Aloe vera-Gel/Pulver mit einem Gehalt an Acemannan von mindestens 10 Gew.-%; und dem lipophilen Stoff oder dem lipophilen Stoffgemisch;
C) Zufügen von maximal 30% (Gew./Vol.) des Pulverkonzentrats zur Trägerlösung zum Herstellen einer Pulverkonzentrat-Trägerlösung;
D) Homogenisieren der Pulverkonzentrat-Trägerlösung unter Bildung einer Mikroemulsion enthaltend Hydrokolloide mit einem durchschnittlichen hydrodynamischen Durchmesser zwischen 800 nm und 1.200 nm, wobei ein Einstellen des pH-Werts auf 4,0 erfolgt,
   wobei während oder nach dem Homogenisieren das Einstellen des pH-Werts auf 4,0 durch Zufügen einer lebensmitteltauglichen Säure, bevorzugt Zitronensäure, Ascorbinsäure, Milchsäure, oder Essigsäure, weiter bevorzugt Zitronensäure oder Ascorbinsäure, besonders bevorzugt Zitronensäure erfolgt.

Bevorzugt ist daher ein Verfahren zur Herstellung einer Mikroemulsion eines lipophilen Stoffes oder lipophilen Stoffgemisches umfassend die Schritte:
A) Bereitstellen einer Trägerlösung umfassend oder bestehend aus einer wässrigen Lösung eines physiologisch verträglichen Elektrolytsalzes und Lecithin mit einem Phosphatidylcholingehalt von mindestens 30%;
   wobei das physiologisch verträgliche Elektrolytsalz ausgewählt wird aus der Gruppe umfassend oder bestehend aus Calciumchlorid, Natriumchlorid, Magnesiumchlorid, und Kaliumchlorid, bevorzugt Calciumchlorid; und/oder
   wobei gereinigtes Wasser, vollentsalztes Wasser, destilliertes Wasser oder Reinstwasser, bevorzugt Reinstwasser verwendet wird;
B) Bereitstellen eines Pulverkonzentrats umfassend oder bestehend aus Gummi arabicum mit einem Gehalt an Arabinogalactanprotein von mindestens 3 Gew.-%, Chitosan mit einem Deacetylierungsgrad von mindestens 70%, Aloe vera-Gel/Pulver mit einem Gehalt an Acemannan von mindestens 10 Gew.-%; und dem lipophilen Stoff oder dem lipophilen Stoffgemisch;
C) Zufügen von maximal 30% (Gew./Vol.) des Pulverkonzentrats zur Trägerlösung zum Herstellen einer Pulverkonzentrat-Trägerlösung;
D) Homogenisieren der Pulverkonzentrat-Trägerlösung unter Bildung einer Mikroemulsion enthaltend Hydrokolloide mit einem durchschnittlichen hydrodynamischen Durchmesser zwischen 800 nm und 1.200 nm, wobei ein Einstellen des pH-Werts auf 4,0 erfolgt,
   wobei während oder nach dem Homogenisieren das Einstellen des pH-Werts auf 4,0 durch Zufügen einer lebensmitteltauglichen Säure, bevorzugt Zitronensäure, Ascorbinsäure, Milchsäure, oder Essigsäure, weiter bevorzugt Zitronensäure oder Ascorbinsäure, besonders bevorzugt Zitronensäure erfolgt.

In bevorzugten Ausführungsformen wird Schritt C) bei einer Temperatur von ≤ 30°C durchgeführt. In bevorzugten Ausführungsformen wird Schritt D) bei einer Temperatur von ≤ 30°C durchgeführt. In bevorzugten Ausführungsformen wird sowohl Schritt C) als auch Schritt D) bei einer Temperatur von ≤ 30°C durchgeführt.

In weiter bevorzugten Ausführungsformen wird Schritt C) bei einer Temperatur von ≤ 15°C durchgeführt. In bevorzugten Ausführungsformen wird Schritt D) bei einer Temperatur von ≤ 15°C durchgeführt. In bevorzugten Ausführungsformen wird sowohl Schritt C) als auch Schritt D) bei einer Temperatur von ≤ 15°C durchgeführt.

In noch weiter bevorzugten Ausführungsformen wird Schritt C) bei einer Temperatur zwischen 5°C und 15°C durchgeführt. In bevorzugten Ausführungsformen wird Schritt D) bei einer Temperatur von zwischen 5°C und 15°C durchgeführt. In bevorzugten Ausführungsformen wird sowohl Schritt C) als auch Schritt D) bei einer Temperatur zwischen 5°C und 15°C durchgeführt.

Beispielsweise können sowohl Schritt C) als auch Schritt D) in einem Eisbad durchgeführt werden, um die Stabilität von temperaturempfindlichen Komponenten sicherzustellen.

In bevorzugten Ausführungsformen wird in Schritt C) ein Maximum von 30% (Gew./Vol.) des Pulverkonzentrates in der Trägerlösung schrittweise und unter kontinuierlichem Rühren gelöst. In weiter bevorzugten Ausführungsformen wird in Schritt C) ein Maximum von 30% (Gew./Vol.) des Pulverkonzentrates in der Trägerlösung schrittweise und unter kontinuierlichem Rühren bei 10.000 U/min gelöst. Die eingesetzte Menge an Pulverkonzentrat hängt von der gewünschten Konzentration des Wirkstoffes/Extraktes in der resultierenden Mikroemulsion ab. Nach der vollständigen Zugabe wird in Schritt D) die Mischung für bevorzugt weitere 15 Minuten bevorzugt bei 10.000 U/min homogenisiert. Der pH-Wert von 4,0 wird dabei bevorzugt während oder nach der Homogenisierung mit einer hierin beschriebenen lebensmitteltauglichen Säure eingestellt

In besonders bevorzugten Ausführungsformen enthält die Mikroemulsion 1 - 5% (Gew./Vol.) lipophilen Stoff oder lipophiles Stoffgemisch, 10 - 25% (Gew./Vol.) Gummi arabicum, 0,5 - 1% (Gew./Vol.) Chitosan, 1 - 5% (Gew./Vol.) Aloe Vera-Gel/Pulver, 2 - 3% (Gew./Vol.) Lecithin und 0,1 - 0,3% (Gew./Vol.) Calciumchlorid.

Bevorzugt ist daher ein Verfahren zur Herstellung einer Mikroemulsion eines lipophilen Stoffes oder lipophilen Stoffgemisches umfassend die Schritte:
A) Bereitstellen einer Trägerlösung umfassend oder bestehend aus einer wässrigen Lösung eines physiologisch verträglichen Elektrolytsalzes und Lecithin mit einem Phosphatidylcholingehalt von mindestens 30%;
B) Bereitstellen eines Pulverkonzentrats umfassend oder bestehend aus Gummi arabicum mit einem Gehalt an Arabinogalactanprotein von mindestens 3 Gew.-%, Chitosan mit einem Deacetylierungsgrad von mindestens 70%, Aloe vera-Gel/Pulver mit einem Gehalt an Acemannan von mindestens 10 Gew.-%; und dem lipophilen Stoff oder dem lipophilen Stoffgemisch;
C) Zufügen von maximal 30% (Gew./Vol.) des Pulverkonzentrats zur Trägerlösung zum Herstellen einer Pulverkonzentrat-Trägerlösung;
D) Homogenisieren der Pulverkonzentrat-Trägerlösung unter Bildung einer Mikroemulsion enthaltend Hydrokolloide mit einem durchschnittlichen hydrodynamischen Durchmesser zwischen 800 nm und 1.200 nm, wobei ein Einstellen des pH-Werts auf 4,0 erfolgt,
   wobei während oder nach dem Homogenisieren das Einstellen des pH-Werts auf 4,0 durch Zufügen einer lebensmitteltauglichen Säure, bevorzugt Zitronensäure, Ascorbinsäure, Milchsäure, oder Essigsäure, weiter bevorzugt Zitronensäure oder Ascorbinsäure, besonders bevorzugt Zitronensäure erfolgt,
wobei die Mikroemulsion 1 - 5% (Gew.Nol.) lipophilen Stoff oder lipophiles Stoffgemisch, 10 - 25% (Gew./Vol.) Gummi arabicum, 0,5 - 1% (Gew./Vol.) Chitosan, 1 - 5% (Gew./Vol.) Aloe Vera-Gel/Pulver, 2 - 3% (Gew./Vol.) Lecithin und 0,1 - 0,3% (Gew./Vol.) Calciumchlorid enthält.

Bevorzugt ist daher ein Verfahren zur Herstellung einer Mikroemulsion eines lipophilen Stoffes oder lipophilen Stoffgemisches umfassend die Schritte:
A) Bereitstellen einer Trägerlösung umfassend oder bestehend aus einer wässrigen Lösung eines physiologisch verträglichen Elektrolytsalzes und Lecithin mit einem Phosphatidylcholingehalt von mindestens 30%;
   wobei das physiologisch verträgliche Elektrolytsalz ausgewählt wird aus der Gruppe umfassend oder bestehend aus Calciumchlorid, Natriumchlorid, Magnesiumchlorid, und Kaliumchlorid, bevorzugt Calciumchlorid; und/oder
   wobei gereinigtes Wasser, vollentsalztes Wasser, destilliertes Wasser oder Reinstwasser, bevorzugt Reinstwasser verwendet wird;
B) Bereitstellen eines Pulverkonzentrats umfassend oder bestehend aus Gummi arabicum mit einem Gehalt an Arabinogalactanprotein von mindestens 3 Gew.-%, Chitosan mit einem Deacetylierungsgrad von mindestens 70%, Aloe vera-Gel/Pulver mit einem Gehalt an Acemannan von mindestens 10 Gew.-%; und dem lipophilen Stoff oder dem lipophilen Stoffgemisch;
C) Zufügen von maximal 30% (Gew./Vol.) des Pulverkonzentrats zur Trägerlösung zum Herstellen einer Pulverkonzentrat-Trägerlösung;
D) Homogenisieren der Pulverkonzentrat-Trägerlösung unter Bildung einer Mikroemulsion enthaltend Hydrokolloide mit einem durchschnittlichen hydrodynamischen Durchmesser zwischen 800 nm und 1.200 nm, wobei ein Einstellen des pH-Werts auf 4,0 erfolgt,
   wobei während oder nach dem Homogenisieren das Einstellen des pH-Werts auf 4,0 durch Zufügen einer lebensmitteltauglichen Säure, bevorzugt Zitronensäure, Ascorbinsäure, Milchsäure, oder Essigsäure, weiter bevorzugt Zitronensäure oder Ascorbinsäure, besonders bevorzugt Zitronensäure erfolgt,
wobei die Mikroemulsion 1 - 5% (Gew.Nol.) lipophilen Stoff oder lipophiles Stoffgemisch, 10 - 25% (Gew./Vol.) Gummi arabicum, 0,5 - 1% (Gew./Vol.) Chitosan, 1 - 5% (Gew./Vol.) Aloe Vera Gel/Pulver, 2 - 3% (Gew./Vol.) Lecithin und 0,1 - 0,3% (Gew./Vol.) Calciumchlorid enthält.

In bevorzugten Ausführungsformen wird Chitosan fungalen Ursprungs verwendet, das aus den Zellwänden bestimmter Pilzarten (z. B. *Mucor spp.* oder *Rhizopus spp*.) gewonnen wird. Das verwendete Chitosan fungalen Ursprungs zeichnet sich insbesondere durch einen gleichmäßigen hohen Grad der Deacetylierung (mindestens 80 %) aus. In bevorzugten Ausführungsformen weist das Chitosan einen Deacetylierungsgrad von mindestens 80%, weiter bevorzugt mindestens 81%, weiter bevorzugt mindestens 82%, weiter bevorzugt mindestens 83%, weiter bevorzugt mindestens 84%, besonders bevorzugt mindestens 85% auf.

In bevorzugten Ausführungsformen wird das Aloe vera-Gel aus dem Inneren der Blätter von Aloe vera barbadensis MILLER verwendet. Dabei wird das Aloe vera-Gel besonders bevorzugt in Form von Aloe vera-Pulver bereitgestellt. In besonders bevorzugten Ausführungsformen weist das Aloe vera-Gel in Form von Pulver, d.h. das Aloe vera-Pulver einen Gehalt an Acemannan von mindestens 13 Gew.-%, weiter bevorzugt mindestens 14 Gew.-% und besonders bevorzugt von mindestens 15 Gew.-% auf. In noch weiter bevorzugten Ausführungsformen wird sprühgetrocknetes Aloe-vera-Pulver mit einem Gehalt an Acemannan von mindestens 13 Gew.-%, weiter bevorzugt mindestens 14 Gew.-% und besonders bevorzugt von mindestens 15 Gew.-% verwendet.

In bevorzugten Ausführungsformen wird Gummi arabicum mit einem Gehalt an AGPs von mindestens 3% vom Typ *Acacia senegal* verwendet. In weiter bevorzugten Ausführungsformen wird sprühgetrocknetes Gummi arabicum mit einem Gehalt an AGPs von mindestens 3% vom Typ *Acacia senegal* verwendet.

In bevorzugten Ausführungsformen wird Lecithin mit einem Phosphatidylcholingehalt von mindestens 40 Gew.-%, weiter von mindestens 45 Gew.-% verwendet. In bevorzugten Ausführungsformen wird Sonnenblumenlecithin oder Sojalecithin verwendet. In weiter bevorzugten Ausführungsformen wird Sonnenblumenlecithin mit einem Phosphatidylcholingehalt von mindestens 40 Gew.-%, weiter von mindestens 45 Gew.-% verwendet. In anderen weiter bevorzugten Ausführungsformen wird Sojalecithin mit einem Phosphatidylcholingehalt von mindestens 40 Gew.-%, weiter von mindestens 45 Gew.-% verwendet.

In bevorzugten Ausführungsformen wird der lipophile Stoff oder das lipophile Stoffgemisch ausgewählt aus der Gruppe umfassend oder besteht aus Hanfextrakte, Cannabinoide (Isolate), Curcuma-Extrakte, Curcuminoide (Isolate), Weihrauch-Extrakte, Boswelliasäuren (Isolate), Coenzym Q10, Ingwer-Extrakte, Ashwagandha-Extrakte, Oligomere Proanthocyanidine, Resveratrol, Vitamin A, Vitamin D, Vitamin E, Vitamin K, omega-3 Fettsäuren aus Fischöl oder Algenöl, Schwarzkümmelöl, Schwarzkümmelextrakte, Artemisia-Extrakte, Extrakte oder Ätherische Öle aus Oregano, Curcuma, Weihrauch, Ingwer, Lavendel, Thymian, Zitrone, Orange, Pfefferminze, Rosmarin, Eukalyptus, Basilikum, Schwarzkümmel, Nelke, Ylang-Ylang, Anis, Kardamon, Salbei, Zitronengras, Wachholderbeeren, Bergamotte, Zitronenverbene, Johanniskraut, Astaxanthin, Spermidin, Quercetin, Fisetin und NAD+.

Bevorzugt ist daher ein Verfahren zur Herstellung einer Mikroemulsion eines lipophilen Stoffes oder lipophilen Stoffgemisches umfassend die Schritte:
A) Bereitstellen einer Trägerlösung umfassend oder bestehend aus einer wässrigen Lösung eines physiologisch verträglichen Elektrolytsalzes und Lecithin mit einem Phosphatidylcholingehalt von mindestens 30%;
B) Bereitstellen eines Pulverkonzentrats umfassend oder bestehend aus Gummi arabicum mit einem Gehalt an Arabinogalactanprotein von mindestens 3 Gew.-%, Chitosan mit einem Deacetylierungsgrad von mindestens 70%, Aloe vera-Gel/Pulver mit einem Gehalt an Acemannan von mindestens 10 Gew.-%; und dem lipophilen Stoff oder dem lipophilen Stoffgemisch;
C) Zufügen von maximal 30% (Gew./Vol.) des Pulverkonzentrats zur Trägerlösung zum Herstellen einer Pulverkonzentrat-Trägerlösung;
D) Homogenisieren der Pulverkonzentrat-Trägerlösung unter Bildung einer Mikroemulsion enthaltend Hydrokolloide mit einem durchschnittlichen hydrodynamischen Durchmesser zwischen 800 nm und 1.200 nm, wobei ein Einstellen des pH-Werts auf 4,0 erfolgt,
   wobei während oder nach dem Homogenisieren das Einstellen des pH-Werts auf 4,0 durch Zufügen einer lebensmitteltauglichen Säure, bevorzugt Zitronensäure, Ascorbinsäure, Milchsäure, oder Essigsäure, weiter bevorzugt Zitronensäure oder Ascorbinsäure, besonders bevorzugt Zitronensäure erfolgt,
wobei der lipophile Stoff oder das lipophile Stoffgemisch ausgewählt werden aus der Gruppe umfassend oder besteht aus Hanfextrakte, Cannabinoide (Isolate), Curcuma-Extrakte, Curcuminoide (Isolate), Weihrauch-Extrakte, Boswelliasäuren (Isolate), Coenzym Q10, Ingwer-Extrakte, Ashwagandha-Extrakte, Oligomere Proanthocyanidine, Resveratrol, Vitamin A, Vitamin D, Vitamin E, Vitamin K, omega-3 Fettsäuren aus Fischöl oder Algenöl, Schwarzkümmelöl, Schwarzkümmelextrakte, Artemisia-Extrakte, Extrakte oder Ätherische Öle aus Oregano, Curcuma, Weihrauch, Ingwer, Lavendel, Thymian, Zitrone, Orange, Pfefferminze, Rosmarin, Eukalyptus, Basilikum, Schwarzkümmel, Nelke, Ylang-Ylang, Anis, Kardamon, Salbei, Zitronengras, Wachholderbeeren, Bergamotte, Zitronenverbene, Johanniskraut, Astaxanthin, Spermidin, Quercetin, Fisetin und NAD+.

Bevorzugt ist daher ein Verfahren zur Herstellung einer Mikroemulsion eines lipophilen Stoffes oder lipophilen Stoffgemisches umfassend die Schritte:
A) Bereitstellen einer Trägerlösung umfassend oder bestehend aus einer wässrigen Lösung eines physiologisch verträglichen Elektrolytsalzes und Lecithin mit einem Phosphatidylcholingehalt von mindestens 30%;
   wobei das physiologisch verträgliche Elektrolytsalz ausgewählt wird aus der Gruppe umfassend oder bestehend aus Calciumchlorid, Natriumchlorid, Magnesiumchlorid, und Kaliumchlorid, bevorzugt Calciumchlorid; und/oder
   wobei gereinigtes Wasser, vollentsalztes Wasser, destilliertes Wasser oder Reinstwasser, bevorzugt Reinstwasser verwendet wird;
B) Bereitstellen eines Pulverkonzentrats umfassend oder bestehend aus Gummi arabicum mit einem Gehalt an Arabinogalactanprotein von mindestens 3 Gew.-%, Chitosan mit einem Deacetylierungsgrad von mindestens 70%, Aloe vera-Gel/Pulver mit einem Gehalt an Acemannan von mindestens 10 Gew.-%; und dem lipophilen Stoff oder dem lipophilen Stoffgemisch;
C) Zufügen von maximal 30% (Gew./Vol.) des Pulverkonzentrats zur Trägerlösung zum Herstellen einer Pulverkonzentrat-Trägerlösung;
D) Homogenisieren der Pulverkonzentrat-Trägerlösung unter Bildung einer Mikroemulsion enthaltend Hydrokolloide mit einem durchschnittlichen hydrodynamischen Durchmesser zwischen 800 nm und 1.200 nm, wobei ein Einstellen des pH-Werts auf 4,0 erfolgt,
   wobei während oder nach dem Homogenisieren das Einstellen des pH-Werts auf 4,0 durch Zufügen einer lebensmitteltauglichen Säure, bevorzugt Zitronensäure, Ascorbinsäure, Milchsäure, oder Essigsäure, weiter bevorzugt Zitronensäure oder Ascorbinsäure, besonders bevorzugt Zitronensäure erfolgt,
wobei der lipophile Stoff oder das lipophile Stoffgemisch ausgewählt werden aus der Gruppe umfassend oder besteht aus Hanfextrakte, Cannabinoide (Isolate), Curcuma-Extrakte, Curcuminoide (Isolate), Weihrauch-Extrakte, Boswelliasäuren (Isolate), Coenzym Q10, Ingwer-Extrakte, Ashwagandha-Extrakte, Oligomere Proanthocyanidine, Resveratrol, Vitamin A, Vitamin D, Vitamin E, Vitamin K, omega-3 Fettsäuren aus Fischöl oder Algenöl, Schwarzkümmelöl, Schwarzkümmelextrakte, Artemisia-Extrakte, Extrakte oder Ätherische Öle aus Oregano, Curcuma, Weihrauch, Ingwer, Lavendel, Thymian, Zitrone, Orange, Pfefferminze, Rosmarin, Eukalyptus, Basilikum, Schwarzkümmel, Nelke, Ylang-Ylang, Anis, Kardamon, Salbei, Zitronengras, Wachholderbeeren, Bergamotte, Zitronenverbene, Johanniskraut, Astaxanthin, Spermidin, Quercetin, Fisetin und NAD+.

Bevorzugt ist daher ein Verfahren zur Herstellung einer Mikroemulsion eines lipophilen Stoffes oder lipophilen Stoffgemisches umfassend die Schritte:
A) Bereitstellen einer Trägerlösung umfassend oder bestehend aus einer wässrigen Lösung eines physiologisch verträglichen Elektrolytsalzes und Lecithin mit einem Phosphatidylcholingehalt von mindestens 30%;
B) Bereitstellen eines Pulverkonzentrats umfassend oder bestehend aus Gummi arabicum mit einem Gehalt an Arabinogalactanprotein von mindestens 3 Gew.-%, Chitosan mit einem Deacetylierungsgrad von mindestens 70%, Aloe vera-Gel/Pulver mit einem Gehalt an Acemannan von mindestens 10 Gew.-%; und dem lipophilen Stoff oder dem lipophilen Stoffgemisch;
C) Zufügen von maximal 30% (Gew./Vol.) des Pulverkonzentrats zur Trägerlösung zum Herstellen einer Pulverkonzentrat-Trägerlösung;
D) Homogenisieren der Pulverkonzentrat-Trägerlösung unter Bildung einer Mikroemulsion enthaltend Hydrokolloide mit einem durchschnittlichen hydrodynamischen Durchmesser zwischen 800 nm und 1.200 nm, wobei ein Einstellen des pH-Werts auf 4,0 erfolgt,
   wobei während oder nach dem Homogenisieren das Einstellen des pH-Werts auf 4,0 durch Zufügen einer lebensmitteltauglichen Säure, bevorzugt Zitronensäure, Ascorbinsäure, Milchsäure, oder Essigsäure, weiter bevorzugt Zitronensäure oder Ascorbinsäure, besonders bevorzugt Zitronensäure erfolgt,
   wobei der lipophile Stoff oder das lipophile Stoffgemisch ausgewählt werden aus der Gruppe umfassend oder besteht aus Hanfextrakte, Cannabinoide (Isolate), Curcuma-Extrakte, Curcuminoide (Isolate), Weihrauch-Extrakte, Boswelliasäuren (Isolate), Coenzym Q10, Ingwer-Extrakte, Ashwagandha-Extrakte, Oligomere Proanthocyanidine, Resveratrol, Vitamin A, Vitamin D, Vitamin E, Vitamin K, omega-3 Fettsäuren aus Fischöl oder Algenöl, Schwarzkümmelöl, Schwarzkümmelextrakte, Artemisia-Extrakte, Extrakte oder Ätherische Öle aus Oregano, Curcuma, Weihrauch, Ingwer, Lavendel, Thymian, Zitrone, Orange, Pfefferminze, Rosmarin, Eukalyptus, Basilikum, Schwarzkümmel, Nelke, Ylang-Ylang, Anis, Kardamon, Salbei, Zitronengras, Wachholderbeeren, Bergamotte, Zitronenverbene, Johanniskraut, Astaxanthin, Spermidin, Quercetin, Fisetin und NAD+,
   wobei die Mikroemulsion 1 - 5% (Gew./Vol.) lipophilen Stoff oder lipophiles Stoffgemisch, 10 - 25% (Gew./Vol.) Gummi arabicum, 0,5 - 1% (Gew./Vol.) Chitosan, 1 - 5% (Gew./Vol.) Aloe Vera-Gel/Pulver, 2 - 3% (Gew./Vol.) Lecithin und 0,1 - 0,3% (Gew./Vol.) Calciumchlorid enthält.

Bevorzugt ist daher ein Verfahren zur Herstellung einer Mikroemulsion eines lipophilen Stoffes oder lipophilen Stoffgemisches umfassend die Schritte:
A) Bereitstellen einer Trägerlösung umfassend oder bestehend aus einer wässrigen Lösung eines physiologisch verträglichen Elektrolytsalzes und Lecithin mit einem Phosphatidylcholingehalt von mindestens 30%;
   wobei das physiologisch verträgliche Elektrolytsalz ausgewählt wird aus der Gruppe umfassend oder bestehend aus Calciumchlorid, Natriumchlorid, Magnesiumchlorid, und Kaliumchlorid, bevorzugt Calciumchlorid; und/oder
   wobei gereinigtes Wasser, vollentsalztes Wasser, destilliertes Wasser oder Reinstwasser, bevorzugt Reinstwasser verwendet wird;
B) Bereitstellen eines Pulverkonzentrats umfassend oder bestehend aus Gummi arabicum mit einem Gehalt an Arabinogalactanprotein von mindestens 3 Gew.-%, Chitosan mit einem Deacetylierungsgrad von mindestens 70%, Aloe vera-Gel/Pulver mit einem Gehalt an Acemannan von mindestens 10 Gew.-%; und dem lipophilen Stoff oder dem lipophilen Stoffgemisch;
C) Zufügen von maximal 30% (Gew./Vol.) des Pulverkonzentrats zur Trägerlösung zum Herstellen einer Pulverkonzentrat-Trägerlösung;
D) Homogenisieren der Pulverkonzentrat-Trägerlösung unter Bildung einer Mikroemulsion enthaltend Hydrokolloide mit einem durchschnittlichen hydrodynamischen Durchmesser zwischen 800 nm und 1.200 nm, wobei ein Einstellen des pH-Werts auf 4,0 erfolgt,
   wobei während oder nach dem Homogenisieren das Einstellen des pH-Werts auf 4,0 durch Zufügen einer lebensmitteltauglichen Säure, bevorzugt Zitronensäure, Ascorbinsäure, Milchsäure, oder Essigsäure, weiter bevorzugt Zitronensäure oder Ascorbinsäure, besonders bevorzugt Zitronensäure erfolgt,

wobei der lipophile Stoff oder das lipophile Stoffgemisch ausgewählt werden aus der Gruppe umfassend oder besteht aus Hanfextrakte, Cannabinoide (Isolate), Curcuma-Extrakte, Curcuminoide (Isolate), Weihrauch-Extrakte, Boswelliasäuren (Isolate), Coenzym Q10, Ingwer-Extrakte, Ashwagandha-Extrakte, Oligomere Proanthocyanidine, Resveratrol, Vitamin A, Vitamin D, Vitamin E, Vitamin K, omega-3 Fettsäuren aus Fischöl oder Algenöl, Schwarzkümmelöl, Schwarzkümmelextrakte, Artemisia-Extrakte, Extrakte oder Ätherische Öle aus Oregano, Curcuma, Weihrauch, Ingwer, Lavendel, Thymian, Zitrone, Orange, Pfefferminze, Rosmarin, Eukalyptus, Basilikum, Schwarzkümmel, Nelke, Ylang-Ylang, Anis, Kardamon, Salbei, Zitronengras, Wachholderbeeren, Bergamotte, Zitronenverbene, Johanniskraut, Astaxanthin, Spermidin, Quercetin, Fisetin und NAD+,
wobei die Mikroemulsion 1 - 5% (Gew./Vol.) lipophilen Stoff oder lipophiles Stoffgemisch, 10 - 25% (Gew./Vol.) Gummi arabicum, 0,5 - 1% (Gew./Vol.) Chitosan, 1 - 5% (Gew./Vol.) Aloe Vera-Gel/Pulver, 2 - 3% (Gew./Vol.) Lecithin und 0,1 - 0,3% (Gew./Vol.) Calciumchlorid enthält.

In bevorzugten Ausführungsformen wird die Trägerlösung hergestellt durch ein Verfahren umfassend die Schritte:
a) Bereitstellen von Wasser, eines physiologisch verträglichen Elektrolytsalzes, bevorzugt Calciumchlorid, und Lecithin mit einem Phosphatidylcholingehalt von mindestens 30 %;
b) Temperieren des Wassers auf eine Temperatur zwischen 30°C - 45°C, bevorzugt 35°C;
c) Zufügen von 2 - 3 % (Gew./Vol.) des Lecithins;
d) Zufügen von 0,1 - 0,3 % (Gew./Vol.) des physiologisch verträglichen Elektrolytsalzes, bevorzugt Calciumchlorid;
e) Entgasen der Trägerlösung.

In bevorzugten Ausführungsformen wird Lecithin mit einem Phosphatidylcholingehalt von mindestens 40 Gew.-%, weiter von mindestens 45 Gew.-% verwendet. In bevorzugten Ausführungsformen wird Sonnenblumenlecithin oder Sojalecithin verwendet. In weiter bevorzugten Ausführungsformen wird Sonnenblumenlecithin mit einem Phosphatidylcholingehalt von mindestens 40 Gew.-%, weiter von mindestens 45 Gew.-% verwendet. In anderen weiter bevorzugten Ausführungsformen wird Sojalecithin mit einem Phosphatidylcholingehalt von mindestens 40 Gew.-%, weiter von mindestens 45 Gew.-% verwendet.

Das physiologisch verträgliche Elektrolytsalz wird bevorzugt ausgewählt aus der Gruppe umfassend oder bestehend aus Calciumchlorid, Natriumchlorid, Magnesiumchlorid, und Kaliumchlorid, bevorzugt Calciumchlorid. In besonders bevorzugten Ausführungsformen ist das physiologisch verträgliche Elektrolytsalz Calciumchlorid. In bevorzugten Ausführungsformen enthält die Trägerlösung das physiologisch verträgliche Elektrolytsalz in einer Konzentration von ca. 9 bis 27 mM.

In bevorzugten Ausführungsformen wird das Wasser zur Herstellung der Mikroemulsion ausgewählt aus gereinigtem Wasser, vollentsalztem Wasser, destilliertem Wasser und Reinstwasser. Am meistens bevorzugt wird Reinstwasser als Lösungsmittel für die Trägerlösung bzw. die Mikroemulsion verwendet.

Nach der Herstellung der Trägerlösung ist eine möglichst vollständige Entgasung in Schritt e) erforderlich, um gelöste Gase zu entfernen, die das Self-Assembling der Hydrokolloide beeinträchtigen könnten. Zu diesem Zweck wird die Trägerlösung bevorzugt in eine Unterdruckkammer überführt und dort einem Unterdruck von weniger als 50 mbar ausgesetzt. Während der Entgasung wird die Lösung bevorzugt kontinuierlich gerührt, um eine gleichmäßige Freisetzung der Gase aus dem gesamten Volumen der Lösung zu gewährleisten. Dieser Prozess wird bevorzugt für eine Dauer von 15 Minuten durchgeführt, wobei die genaue Zeitdauer je nach Gesamtvolumen der Lösung angepasst werden muss.

Alternativ kann die Lösung mittels Ultraschalls im Eisbad entgast werden, wobei darauf zu achten ist, dass die Ultraschallintensität nicht zu hoch gewählt wird. Das Eisbad dient dazu, die Temperatur während des Prozesses niedrig zu halten und hitzelabile Komponenten zu schützen. Ein zu hoher Energieeintrag kann empfindliche Komponenten der Trägerlösung beschädigen, abbauen oder die elektrostatischen Bindungen zwischen ihnen beeinträchtigen.

In weiter bevorzugten Ausführungsformen wird die Trägerlösung hergestellt durch ein Verfahren umfassend die Schritte:
a) Bereitstellen von Wasser, eines physiologisch verträglichen Elektrolytsalzes, bevorzugt Calciumchlorid, und Lecithin mit einem Phosphatidylcholingehalt von mindestens 30 %;
b) Temperieren des Wassers auf eine Temperatur zwischen 30°C - 45°C, bevorzugt 35°C;
c) Zufügen von 2 - 3 % (Gew./Vol.) des Lecithins;
d) Zufügen von 0,1 - 0,3 % (Gew./Vol.) des physiologisch verträglichen Elektrolytsalzes, bevorzugt Calciumchlorid;
e) Entgasen der Trägerlösung,
wobei das Wasser ausgewählt wird aus gereinigtem Wasser, vollentsalztem Wasser, destilliertem Wasser und Reinstwasser.

In noch weiter bevorzugten Ausführungsformen wird die Trägerlösung hergestellt durch ein Verfahren umfassend die Schritte:
a) Bereitstellen von Wasser, eines physiologisch verträglichen Elektrolytsalzes, bevorzugt Calciumchlorid, und Lecithin mit einem Phosphatidylcholingehalt von mindestens 30 %;
b) Temperieren des Wassers auf eine Temperatur zwischen 30°C - 45°C, bevorzugt 35°C;
c) Zufügen von 2 - 3 % (Gew./Vol.) des Lecithins;
d) Zufügen von 0,1 - 0,3 % (Gew./Vol.) des physiologisch verträglichen Elektrolytsalzes, bevorzugt Calciumchlorid;
e) Entgasen der Trägerlösung,
wobei das Wasser ausgewählt wird aus Reinstwasser.

In weiter bevorzugten Ausführungsformen wird die Trägerlösung hergestellt durch ein Verfahren umfassend die Schritte:
a) Bereitstellen von Wasser, eines physiologisch verträglichen Elektrolytsalzes, bevorzugt Calciumchlorid, und Lecithin mit einem Phosphatidylcholingehalt von mindestens 30 %;
b) Temperieren des Wassers auf eine Temperatur zwischen 30°C - 45°C, bevorzugt 35°C;
c) Zufügen von 2 - 3 % (Gew./Vol.) des Lecithins;
d) Zufügen von 0,1 - 0,3 % (Gew./Vol.) des physiologisch verträglichen Elektrolytsalzes, bevorzugt Calciumchlorid;
e) Entgasen der Trägerlösung,
wobei das physiologisch verträgliche Elektrolytsalz ausgewählt wird aus der Gruppe umfassend oder bestehend aus Calciumchlorid, Natriumchlorid, Magnesiumchlorid, und Kaliumchlorid, bevorzugt Calciumchlorid.

In weiter bevorzugten Ausführungsformen wird die Trägerlösung hergestellt durch ein Verfahren umfassend die Schritte:
a) Bereitstellen von Wasser, eines physiologisch verträglichen Elektrolytsalzes, bevorzugt Calciumchlorid, und Lecithin mit einem Phosphatidylcholingehalt von mindestens 30 %;
b) Temperieren des Wassers auf eine Temperatur zwischen 30°C - 45°C, bevorzugt 35°C;
c) Zufügen von 2 - 3 % (Gew./Vol.) des Lecithins;
d) Zufügen von 0,1 - 0,3 % (Gew./Vol.) des physiologisch verträglichen Elektrolytsalzes, bevorzugt Calciumchlorid;
e) Entgasen der Trägerlösung,

wobei das physiologisch verträgliche Elektrolytsalz ausgewählt wird aus der Gruppe umfassend oder bestehend aus Calciumchlorid, Natriumchlorid, Magnesiumchlorid, und Kaliumchlorid, bevorzugt Calciumchlorid, und
wobei das Wasser ausgewählt wird aus gereinigtem Wasser, vollentsalztem Wasser, destilliertem Wasser und Reinstwasser.

In noch weiter bevorzugten Ausführungsformen wird die Trägerlösung hergestellt durch ein Verfahren umfassend die Schritte:
a) Bereitstellen von Wasser, eines physiologisch verträglichen Elektrolytsalzes, bevorzugt Calciumchlorid, und Lecithin mit einem Phosphatidylcholingehalt von mindestens 30 %;
b) Temperieren des Wassers auf eine Temperatur zwischen 30°C - 45°C, bevorzugt 35°C;
c) Zufügen von 2 - 3 % (Gew./Vol.) des Lecithins;
d) Zufügen von 0,1 - 0,3 % (Gew./Vol.) des physiologisch verträglichen Elektrolytsalzes, bevorzugt Calciumchlorid;
e) Entgasen der Trägerlösung,

wobei das physiologisch verträgliche Elektrolytsalz ausgewählt wird aus der Gruppe umfassend oder bestehend aus Calciumchlorid, Natriumchlorid, Magnesiumchlorid, und Kaliumchlorid, bevorzugt Calciumchlorid, und
wobei das Wasser ausgewählt wird aus gereinigtem Wasser, vollentsalztem Wasser, destilliertem Wasser und Reinstwasser.

In noch weiter bevorzugten Ausführungsformen wird die Trägerlösung hergestellt durch ein Verfahren umfassend die Schritte:
a) Bereitstellen von Wasser, eines physiologisch verträglichen Elektrolytsalzes, bevorzugt Calciumchlorid, und Lecithin mit einem Phosphatidylcholingehalt von mindestens 30 %;
b) Temperieren des Wassers auf eine Temperatur zwischen 30°C - 45°C, bevorzugt 35°C;
c) Zufügen von 2 - 3 % (Gew./Vol.) des Lecithins;
d) Zufügen von 0,1 - 0,3 % (Gew./Vol.) des physiologisch verträglichen Elektrolytsalzes, bevorzugt Calciumchlorid;
e) Entgasen der Trägerlösung,
wobei Lecithin mit einem Phosphatidylcholingehalt von mindestens 40 Gew.-%, weiter von mindestens 45 Gew.-% verwendet wird.

In weiter bevorzugten Ausführungsformen wird die Trägerlösung hergestellt durch ein Verfahren umfassend die Schritte:
a) Bereitstellen von Wasser, eines physiologisch verträglichen Elektrolytsalzes, bevorzugt Calciumchlorid, und Lecithin mit einem Phosphatidylcholingehalt von mindestens 30 %;
b) Temperieren des Wassers auf eine Temperatur zwischen 30°C - 45°C, bevorzugt 35°C;
c) Zufügen von 2 - 3 % (Gew./Vol.) des Lecithins;
d) Zufügen von 0,1 - 0,3 % (Gew./Vol.) des physiologisch verträglichen Elektrolytsalzes, bevorzugt Calciumchlorid;
e) Entgasen der Trägerlösung,

wobei das physiologisch verträgliche Elektrolytsalz ausgewählt wird aus der Gruppe umfassend oder bestehend aus Calciumchlorid, Natriumchlorid, Magnesiumchlorid, und Kaliumchlorid, bevorzugt Calciumchlorid,
wobei Lecithin mit einem Phosphatidylcholingehalt von mindestens 40 Gew.-%, weiter von mindestens 45 Gew.-% verwendet wird.

In weiter bevorzugten Ausführungsformen wird die Trägerlösung hergestellt durch ein Verfahren umfassend die Schritte:
a) Bereitstellen von Wasser, eines physiologisch verträglichen Elektrolytsalzes, bevorzugt Calciumchlorid, und Lecithin mit einem Phosphatidylcholingehalt von mindestens 30 %;
b) Temperieren des Wassers auf eine Temperatur zwischen 30°C - 45°C, bevorzugt 35°C;
c) Zufügen von 2 - 3 % (Gew./Vol.) des Lecithins;
d) Zufügen von 0,1 - 0,3 % (Gew./Vol.) des physiologisch verträglichen Elektrolytsalzes, bevorzugt Calciumchlorid;
e) Entgasen der Trägerlösung,

wobei das physiologisch verträgliche Elektrolytsalz ausgewählt wird aus der Gruppe umfassend oder bestehend aus Calciumchlorid, Natriumchlorid, Magnesiumchlorid, und Kaliumchlorid, bevorzugt Calciumchlorid, und
wobei das Wasser ausgewählt wird aus gereinigtem Wasser, vollentsalztem Wasser, destilliertem Wasser und Reinstwasser,
wobei Lecithin mit einem Phosphatidylcholingehalt von mindestens 40 Gew.-%, weiter von mindestens 45 Gew.-% verwendet wird.

In noch weiter bevorzugten Ausführungsformen wird die Trägerlösung hergestellt durch ein Verfahren umfassend die Schritte:
a) Bereitstellen von Wasser, eines physiologisch verträglichen Elektrolytsalzes, bevorzugt Calciumchlorid, und Lecithin mit einem Phosphatidylcholingehalt von mindestens 30 %;
b) Temperieren des Wassers auf eine Temperatur zwischen 30°C - 45°C, bevorzugt 35°C;
c) Zufügen von 2 - 3 % (Gew./Vol.) des Lecithins;
d) Zufügen von 0,1 - 0,3 % (Gew./Vol.) des physiologisch verträglichen Elektrolytsalzes, bevorzugt Calciumchlorid;
e) Entgasen der Trägerlösung,

wobei das physiologisch verträgliche Elektrolytsalz ausgewählt wird aus der Gruppe umfassend oder bestehend aus Calciumchlorid, Natriumchlorid, Magnesiumchlorid, und Kaliumchlorid, bevorzugt Calciumchlorid, und
wobei das Wasser ausgewählt wird aus gereinigtem Wasser, vollentsalztem Wasser, destilliertem Wasser und Reinstwasser,
wobei Lecithin mit einem Phosphatidylcholingehalt von mindestens 40 Gew.-%, weiter von mindestens 45 Gew.-% verwendet wird.

In noch weiter bevorzugten Ausführungsformen wird die Trägerlösung hergestellt durch ein Verfahren umfassend die Schritte:
a) Bereitstellen von Wasser, eines physiologisch verträglichen Elektrolytsalzes, bevorzugt Calciumchlorid, und Lecithin mit einem Phosphatidylcholingehalt von mindestens 30 %;
b) Temperieren des Wassers auf eine Temperatur zwischen 30°C - 45°C, bevorzugt 35°C;
c) Zufügen von 2 - 3 % (Gew./Vol.) des Lecithins;
d) Zufügen von 0,1 - 0,3 % (Gew./Vol.) des physiologisch verträglichen Elektrolytsalzes, bevorzugt Calciumchlorid;
e) Entgasen der Trägerlösung,
wobei Lecithin ausgewählt wird aus Sonnenblumenlecithin mit einem Phosphatidylcholingehalt von mindestens 40 Gew.-%, weiter von mindestens 45 Gew.-% oder Sojalecithin mit einem Phosphatidylcholingehalt von mindestens 40 Gew.-%, weiter von mindestens 45 Gew.-%.

In weiter bevorzugten Ausführungsformen wird die Trägerlösung hergestellt durch ein Verfahren umfassend die Schritte:
a) Bereitstellen von Wasser, eines physiologisch verträglichen Elektrolytsalzes, bevorzugt Calciumchlorid, und Lecithin mit einem Phosphatidylcholingehalt von mindestens 30 %;
b) Temperieren des Wassers auf eine Temperatur zwischen 30°C - 45°C, bevorzugt 35°C;
c) Zufügen von 2 - 3 % (Gew./Vol.) des Lecithins;
d) Zufügen von 0,1 - 0,3 % (Gew./Vol.) des physiologisch verträglichen Elektrolytsalzes, bevorzugt Calciumchlorid;
e) Entgasen der Trägerlösung,

wobei das physiologisch verträgliche Elektrolytsalz ausgewählt wird aus der Gruppe umfassend oder bestehend aus Calciumchlorid, Natriumchlorid, Magnesiumchlorid, und Kaliumchlorid, bevorzugt Calciumchlorid,
wobei Lecithin ausgewählt wird aus Sonnenblumenlecithin mit einem Phosphatidylcholingehalt von mindestens 40 Gew.-%, weiter von mindestens 45 Gew.-% oder Sojalecithin mit einem Phosphatidylcholingehalt von mindestens 40 Gew.-%, weiter von mindestens 45 Gew.-%.

In weiter bevorzugten Ausführungsformen wird die Trägerlösung hergestellt durch ein Verfahren umfassend die Schritte:
a) Bereitstellen von Wasser, eines physiologisch verträglichen Elektrolytsalzes, bevorzugt Calciumchlorid, und Lecithin mit einem Phosphatidylcholingehalt von mindestens 30 %;
b) Temperieren des Wassers auf eine Temperatur zwischen 30°C - 45°C, bevorzugt 35°C;
c) Zufügen von 2 - 3 % (Gew./Vol.) des Lecithins;
d) Zufügen von 0,1 - 0,3 % (Gew./Vol.) des physiologisch verträglichen Elektrolytsalzes, bevorzugt Calciumchlorid;
e) Entgasen der Trägerlösung,

wobei das physiologisch verträgliche Elektrolytsalz ausgewählt wird aus der Gruppe umfassend oder bestehend aus Calciumchlorid, Natriumchlorid, Magnesiumchlorid, und Kaliumchlorid, bevorzugt Calciumchlorid, und
wobei das Wasser ausgewählt wird aus gereinigtem Wasser, vollentsalztem Wasser, destilliertem Wasser und Reinstwasser,
wobei Lecithin ausgewählt wird aus Sonnenblumenlecithin mit einem Phosphatidylcholingehalt von mindestens 40 Gew.-%, weiter von mindestens 45 Gew.-% oder Sojalecithin mit einem Phosphatidylcholingehalt von mindestens 40 Gew.-%, weiter von mindestens 45 Gew.-%.

In noch weiter bevorzugten Ausführungsformen wird die Trägerlösung hergestellt durch ein Verfahren umfassend die Schritte:
a) Bereitstellen von Wasser, eines physiologisch verträglichen Elektrolytsalzes, bevorzugt Calciumchlorid, und Lecithin mit einem Phosphatidylcholingehalt von mindestens 30 %;
b) Temperieren des Wassers auf eine Temperatur zwischen 30°C - 45°C, bevorzugt 35°C;
c) Zufügen von 2 - 3 % (Gew./Vol.) des Lecithins;
d) Zufügen von 0,1 - 0,3 % (Gew./Vol.) des physiologisch verträglichen Elektrolytsalzes, bevorzugt Calciumchlorid;
e) Entgasen der Trägerlösung,

wobei das physiologisch verträgliche Elektrolytsalz ausgewählt wird aus der Gruppe umfassend oder bestehend aus Calciumchlorid, Natriumchlorid, Magnesiumchlorid, und Kaliumchlorid, bevorzugt Calciumchlorid, und
wobei das Wasser ausgewählt wird aus gereinigtem Wasser, vollentsalztem Wasser, destilliertem Wasser und Reinstwasser,
wobei Lecithin ausgewählt wird aus Sonnenblumenlecithin mit einem Phosphatidylcholingehalt von mindestens 40 Gew.-%, weiter von mindestens 45 Gew.-% oder Sojalecithin mit einem Phosphatidylcholingehalt von mindestens 40 Gew.-%, weiter von mindestens 45 Gew.-%.

In bevorzugten Ausführungsformen wird das Pulverkonzentrat hergestellt durch ein Verfahren umfassend die Schritte:
i) Bereitstellen von Lösungsmittel, Gummi arabicum mit einem Gehalt an Arabinogalactanprotein von mindestens 3 Gew.-%, Chitosan mit einem Deacetylierungsgrad von mindestens 75%, Aloe vera-Gel/Pulver mit einem Gehalt an Acemannan von mindestens 10 Gew.-%; und lipophilen Stoff oder lipophilen Stoffgemisch;
ii) Lösen oder Dispergieren von 2 - 10 % (Gew./Vol.) des lipophilen Stoffs oder lipophilen Stoffgemischs im Lösungsmittel und Homogeniseren;
iii) Zufügen von 20 - 50 % (Gew./Vol.) des Gummi arabicums;
iv) Zufügen von 1 - 5 % (Gew./Vol.) des Chitosans;
v) Zufügen von 2 - 10 % (Gew./Vol.) der Aloe vera-Gel/Pulver;
vi) Homogenisieren;
vii) optionales Entgasen; und
viii) Entfernen des Lösungsmittels.

Besonders bevorzugt ist das Lösungsmittel Ethanol, insbesondere lebensmitteltaugliches Ethanol.

In bevorzugten Ausführungsformen wird Chitosan fungalen Ursprungs verwendet, das aus den Zellwänden bestimmter Pilzarten (z. B. *Mucor spp.* oder *Rhizopus spp*.) gewonnen wird. Das verwendete Chitosan fungalen Ursprungs zeichnet sich insbesondere durch einen gleichmäßigen hohen Grad der Deacetylierung (mindestens 80 %) aus. In bevorzugten Ausführungsformen weist das Chitosan einen Deacetylierungsgrad von mindestens 80%, weiter bevorzugt mindestens 81%, weiter bevorzugt mindestens 82%, weiter bevorzugt mindestens 83%, weiter bevorzugt mindestens 84%, besonders bevorzugt mindestens 85% auf.

In bevorzugten Ausführungsformen wird das Aloe vera-Gel aus dem Inneren der Blätter von Aloe vera barbadensis MILLER verwendet. Dabei wird das Aloe vera-Gel besonders bevorzugt in Form von Aloe vera-Pulver bereitgestellt. In besonders bevorzugten Ausführungsformen weist das Aloe vera-Gel in Form von Pulver, d.h. das Aloe vera-Pulver einen Gehalt an Acemannan von mindestens 13 Gew.-%, weiter bevorzugt mindestens 14 Gew.-% und besonders bevorzugt von mindestens 15 Gew.-% auf. In noch weiter bevorzugten Ausführungsformen wird sprühgetrocknetes Aloe-vera-Pulver mit einem Gehalt an Acemannan von mindestens 13 Gew.-%, weiter bevorzugt mindestens 14 Gew.-% und besonders bevorzugt von mindestens 15 Gew.-% verwendet.

In bevorzugten Ausführungsformen wird Gummi arabicum mit einem Gehalt an AGPs von mindestens 3% vom Typ *Acacia senegal* verwendet. In weiter bevorzugten Ausführungsformen wird sprühgetrocknetes Gummi arabicum mit einem Gehalt an AGPs von mindestens 3% vom Typ *Acacia senegal* verwendet.

In bevorzugten Ausführungsformen wird der lipophile Stoff oder das lipophile Stoffgemisch ausgewählt werden aus der Gruppe umfassend oder besteht aus Hanfextrakte, Cannabinoide (Isolate), Curcuma-Extrakte, Curcuminoide (Isolate), Weihrauch-Extrakte, Boswelliasäuren (Isolate), Coenzym Q10, Ingwer-Extrakte, Ashwagandha-Extrakte, Oligomere Proanthocyanidine, Resveratrol, Vitamin A, Vitamin D, Vitamin E, Vitamin K, omega-3 Fettsäuren aus Fischöl oder Algenöl, Schwarzkümmelöl, Schwarzkümmelextrakte, Artemisia-Extrakte, Extrakte oder Ätherische Öle aus Oregano, Curcuma, Weihrauch, Ingwer, Lavendel, Thymian, Zitrone, Orange, Pfefferminze, Rosmarin, Eukalyptus, Basilikum, Schwarzkümmel, Nelke, Ylang-Ylang, Anis, Kardamon, Salbei, Zitronengras, Wachholderbeeren, Bergamotte, Zitronenverbene, Johanniskraut, Astaxanthin, Spermidin, Quercetin, Fisetin und NAD+.

In bevorzugten Ausführungsformen wird das Pulverkonzentrat hergestellt durch ein Verfahren umfassend die Schritte:
i) Bereitstellen von Lösungsmittel, Gummi arabicum mit einem Gehalt an Arabinogalactanprotein von mindestens 3 Gew.-%, Chitosan mit einem Deacetylierungsgrad von mindestens 75%, Aloe vera-Gel/Pulver mit einem Gehalt an Acemannan von mindestens 10 Gew.-%; und lipophilen Stoff oder lipophilen Stoffgemisch;
ii) Lösen oder Dispergieren von 2 - 10 % (Gew./Vol.) des lipophilen Stoffs oder lipophilen Stoffgemischs im Lösungsmittel und Homogenisieren;
iii) Zufügen von 20 - 50 % (Gew./Vol.) des Gummi arabicums;
iv) Zufügen von 1 - 5 % (Gew./Vol.) des Chitosans;
v) Zufügen von 2 - 10 % (Gew./Vol.) des Aloe vera-Gels/Pulvers;
vi) Homogenisieren;
vii) optionales Entgasen; und
viii) Entfernen des Lösungsmittels,
wobei Chitosan einen Deacetylierungsgrad von mindestens 80%, weiter bevorzugt mindestens 81%, weiter bevorzugt mindestens 82%, weiter bevorzugt mindestens 83%, weiter bevorzugt mindestens 84%, besonders bevorzugt mindestens 85% aufweist.

Besonders bevorzugt ist das Lösungsmittel Ethanol, insbesondere lebensmitteltaugliches Ethanol.

In bevorzugten Ausführungsformen wird das Pulverkonzentrat hergestellt durch ein Verfahren umfassend die Schritte:
i) Bereitstellen von Lösungsmittel, Gummi arabicum mit einem Gehalt an Arabinogalactanprotein von mindestens 3 Gew.-%, Chitosan mit einem Deacetylierungsgrad von mindestens 75%, Aloe vera-Gel/Pulver mit einem Gehalt an Acemannan von mindestens 10 Gew.-%; und lipophilen Stoff oder lipophilen Stoffgemisch;
ii) Lösen oder Dispergieren von 2 - 10 % (Gew./Vol.) des lipophilen Stoffs oder lipophilen Stoffgemischs im Lösungsmittel und Homogenisieren;
iii) Zufügen von 20 - 50 % (Gew./Vol.) des Gummi arabicums;
iv) Zufügen von 1 - 5 % (Gew./Vol.) des Chitosans;
v) Zufügen von 2 - 10 % (Gew./Vol.) des Aloe vera-Gels/Pulver;
vi) Homogenisieren;
vii) optionales Entgasen; und
viii) Entfernen des Lösungsmittels,
wobei das Aloe vera-Gel/Pulver in Form von sprühgetrocknetem Aloe-vera-Pulver mit einem Gehalt an Acemannan von mindestens 13 Gew.-%, weiter bevorzugt mindestens 14 Gew.-% und besonders bevorzugt von mindestens 15 Gew.-% verwendet wird.

Besonders bevorzugt ist das Lösungsmittel Ethanol, insbesondere lebensmitteltaugliches Ethanol.

In bevorzugten Ausführungsformen wird das Pulverkonzentrat hergestellt durch ein Verfahren umfassend die Schritte:
i) Bereitstellen von Lösungsmittel, Gummi arabicum mit einem Gehalt an Arabinogalactanprotein von mindestens 3 Gew.-%, Chitosan mit einem Deacetylierungsgrad von mindestens 75%, Aloe vera-Gel/Pulver mit einem Gehalt an Acemannan von mindestens 10 Gew.-%; und lipophilen Stoff oder lipophilen Stoffgemisch;
ii) Lösen oder Dispergieren von 2 - 10 % (Gew./Vol.) des lipophilen Stoffs oder lipophilen Stoffgemischs im Lösungsmittel und Homogenisieren;
iii) Zufügen von 20 - 50 % (Gew./Vol.) des Gummi arabicums;
iv) Zufügen von 1 - 5 % (Gew./Vol.) des Chitosans;
v) Zufügen von 2 - 10 % (Gew./Vol.) des Aloe vera-Gels/Pulvers;
vi) Homogenisieren;
vii) optionales Entgasen; und
viii) Entfernen des Lösungsmittels,

wobei Chitosan einen Deacetylierungsgrad von mindestens 80%, weiter bevorzugt mindestens 81%, weiter bevorzugt mindestens 82%, weiter bevorzugt mindestens 83%, weiter bevorzugt mindestens 84%, besonders bevorzugt mindestens 85% aufweist,
wobei das Aloe vera-Gel/Pulver in Form von sprühgetrocknetem Aloe-vera-Pulver mit einem Gehalt an Acemannan von mindestens 13 Gew.-%, weiter bevorzugt mindestens 14 Gew.-% und besonders bevorzugt von mindestens 15 Gew.-% verwendet wird.

Besonders bevorzugt ist das Lösungsmittel Ethanol, insbesondere lebensmitteltaugliches Ethanol.

In bevorzugten Ausführungsformen wird das Pulverkonzentrat hergestellt durch ein Verfahren umfassend die Schritte:
i) Bereitstellen von Lösungsmittel, Gummi arabicum mit einem Gehalt an Arabinogalactanprotein von mindestens 3 Gew.-%, Chitosan mit einem Deacetylierungsgrad von mindestens 75%, Aloe vera-Gel/Pulver mit einem Gehalt an Acemannan von mindestens 10 Gew.-%; und lipophilen Stoff oder lipophilen Stoffgemisch;
ii) Lösen oder Dispergieren von 2 - 10 % (Gew./Vol.) des lipophilen Stoffs oder lipophilen Stoffgemischs im Lösungsmittel und Homogenisieren;
iii) Zufügen von 20 - 50 % (Gew./Vol.) des Gummi arabicums;
iv) Zufügen von 1 - 5 % (Gew./Vol.) des Chitosans;
v) Zufügen von 2 - 10 % (Gew./Vol.) des Aloe vera-Gels/Pulvers;
vi) Homogenisieren;
vii) optionales Entgasen; und
viii) Entfernen des Lösungsmittels,
wobei Gummi arabicum in Form von sprühgetrocknetem Gummi arabicum mit einem Gehalt an AGPs von mindestens 3% vom Typ *Acacia senegal* verwendet wird.

Besonders bevorzugt ist das Lösungsmittel Ethanol, insbesondere lebensmitteltaugliches Ethanol.

In bevorzugten Ausführungsformen wird das Pulverkonzentrat hergestellt durch ein Verfahren umfassend die Schritte:
i) Bereitstellen von Lösungsmittel, Gummi arabicum mit einem Gehalt an Arabinogalactanprotein von mindestens 3 Gew.-%, Chitosan mit einem Deacetylierungsgrad von mindestens 75%, Aloe vera-Gel/Pulver mit einem Gehalt an Acemannan von mindestens 10 Gew.-%; und lipophilen Stoff oder lipophilen Stoffgemisch;
ii) Lösen oder Dispergieren von 2 - 10 % (Gew./Vol.) des lipophilen Stoffs oder lipophilen Stoffgemischs im Lösungsmittel und Homogenisieren;
iii) Zufügen von 20 - 50 % (Gew./Vol.) des Gummi arabicums;
iv) Zufügen von 1 - 5 % (Gew./Vol.) des Chitosans;
v) Zufügen von 2 - 10 % (Gew./Vol.) des Aloe vera-Gels/Pulvers;
vi) Homogenisieren;
vii) optionales Entgasen; und
viii) Entfernen des Lösungsmittels,
wobei Gummi arabicum in Form von sprühgetrocknetem Gummi arabicum mit einem Gehalt an AGPs von mindestens 3% vom Typ *Acacia senegal* verwendet wird, wobei Chitosan einen Deacetylierungsgrad von mindestens 80%, weiter bevorzugt mindestens 81%, weiter bevorzugt mindestens 82%, weiter bevorzugt mindestens 83%, weiter bevorzugt mindestens 84%, besonders bevorzugt mindestens 85% aufweist.

Besonders bevorzugt ist das Lösungsmittel Ethanol, insbesondere lebensmitteltaugliches Ethanol.

In bevorzugten Ausführungsformen wird das Pulverkonzentrat hergestellt durch ein Verfahren umfassend die Schritte:
i) Bereitstellen von Lösungsmittel, Gummi arabicum mit einem Gehalt an Arabinogalactanprotein von mindestens 3 Gew.-%, Chitosan mit einem Deacetylierungsgrad von mindestens 75%, Aloe vera-Gel/Pulver mit einem Gehalt an Acemannan von mindestens 10 Gew.-%; und lipophilen Stoff oder lipophilen Stoffgemisch;
ii) Lösen oder Dispergieren von 2 - 10 % (Gew./Vol.) des lipophilen Stoffs oder lipophilen Stoffgemischs im Lösungsmittel und Homogenisieren;
iii) Zufügen von 20 - 50 % (Gew./Vol.) des Gummi arabicums;
iv) Zufügen von 1 - 5 % (Gew./Vol.) des Chitosans;
v) Zufügen von 2 - 10 % (Gew./Vol.) des Aloe vera-Gels/Pulvers;
vi) Homogenisieren;
vii) optionales Entgasen; und
viii) Entfernen des Lösungsmittels,

wobei Gummi arabicum in Form von sprühgetrocknetem Gummi arabicum mit einem Gehalt an AGPs von mindestens 3% vom Typ *Acacia senegal* verwendet wird,
wobei das Aloe vera-Gel/Pulver in Form von sprühgetrocknetem Aloe-vera-Pulver mit einem Gehalt an Acemannan von mindestens 13 Gew.-%, weiter bevorzugt mindestens 14 Gew.-% und besonders bevorzugt von mindestens 15 Gew.-% verwendet wird.

Besonders bevorzugt ist das Lösungsmittel Ethanol, insbesondere lebensmitteltaugliches Ethanol.

In bevorzugten Ausführungsformen wird das Pulverkonzentrat hergestellt durch ein Verfahren umfassend die folgenden Schritte:
i) Bereitstellen von Lösungsmittel, Gummi arabicum mit einem Gehalt an Arabinogalactanprotein von mindestens 3 Gew.-%, Chitosan mit einem Deacetylierungsgrad von mindestens 75%, Aloe vera-Gel/Pulver mit einem Gehalt an Acemannan von mindestens 10 Gew.-%; und lipophilen Stoff oder lipophilen Stoffgemisch;
ii) Lösen oder Dispergieren von 2 - 10 % (Gew./Vol.) des lipophilen Stoffs oder lipophilen Stoffgemischs im Lösungsmittel und Homogenisieren;
iii) Zufügen von 20 - 50 % (Gew./Vol.) des Gummi arabicums;
iv) Zufügen von 1 - 5 % (Gew./Vol.) des Chitosans;
v) Zufügen von 2 - 10 % (Gew./Vol.) des Aloe vera-Gels/Pulvers;
vi) Homogenisieren;
vii) optionales Entgasen; und
viii) Entfernen des Lösungsmittels,

wobei Gummi arabicum in Form von sprühgetrocknetem Gummi arabicum mit einem Gehalt an AGPs von mindestens 3% vom Typ *Acacia senegal* verwendet wird,
wobei Chitosan einen Deacetylierungsgrad von mindestens 80%, weiter bevorzugt mindestens 81%, weiter bevorzugt mindestens 82%, weiter bevorzugt mindestens 83%, weiter bevorzugt mindestens 84%, besonders bevorzugt mindestens 85% aufweist,
wobei das Aloe vera-Gel/Pulver in Form von sprühgetrocknetem Aloe-vera-Pulver mit einem Gehalt an Acemannan von mindestens 13 Gew.-%, weiter bevorzugt mindestens 14 Gew.-% und besonders bevorzugt von mindestens 15 Gew.-% verwendet wird.

Besonders bevorzugt ist das Lösungsmittel Ethanol, insbesondere lebensmitteltaugliches Ethanol.

Die genauen Mengen an GA, Chitosan und Aloe Vera können wirkstoffspezifisch in Reihenversuchen ermittelt werden, um eine optimale Stabilisierung zu erreichen. Zu wenig GA führt dazu, dass die Wirkstoffe ausfallen, wenn das GA-Wirkstoff-Pulver im nachfolgenden Schritt in Wasser gelöst wird. Dies ist darauf zurückzuführen, dass die AGPs aus GA an der Grenzfläche eine stabilisierende Schutzschicht um hydrophobe Wirkstoffpartikel bilden. Fehlt es an einer ausreichenden Menge an GA, bleibt die Schutzschicht unvollständig, wodurch hydrophobe Wirkstoffe unkontrolliert aggregieren und schließlich ausfallen können. Eine übermäßige Zugabe von GA hingegen erhöht die Viskosität der Lösung signifikant, was das Self-Assembling der Hydrokolloide behindert. Dieses Self-Assembling, ein zentraler Prozess bei der Bildung von Mizellen, erfordert ein möglichst niedervisköses Medium, das ausreichend Bewegungsfreiheit für die Moleküle bietet. Eine hochvisköse Lösung schränkt diese Dynamik ein und führt zu einer ineffizienten Bildung stabiler Mizellenstrukturen.

Besonders vorteilhafte und optimale Ergebnisse konnten mit den folgenden Verhältnissen erzielt werden:
Lipophiler Stoff/Lipophiles Stoffgemisch (Wirkstoff/Extrakt) zu Gummi Arabicum (GA): zwischen 1:8 und 1:15. (Maximum bei 23% (Gew./Vol.) des Volumens der Mikroemulsion)
Lipophiler Stoff/Lipophiles Stoffgemisch (Wirkstoff/Extrakt) zu Chitosan: zwischen 1:0.5 und 1:2. (Maximum bei 2.5% (w/v) des Volumens der Mikroemulsion)
Lipophiler Stoff/Lipophiles Stoffgemisch (Wirkstoff/Extrakt) zu Aloe Vera-Gel/Pulver: zwischen 1:1 und 1:5. (Maximum bei 10% (w/v) des Volumens der Mikroemulsion)
Maximum von Gummi Arabicum, Chitosan und Aloe Vera-Gel/Pulver: 30% (Gew./Vol.) des Volumens der Mikroemulsion.

Die Schritte ii) bis viii) werden bevorzugt unter gekühlten Bedingungen durchgeführt, wobei die Lösung z.B. während des gesamten Prozesses in einem Eisbad gehalten wird. Die Kühlung ist notwendig, um temperaturempfindliche Komponenten zu schützen, insbesondere bei der Ultraschallbehandlung, da hier thermische Energie freigesetzt wird.

Schritt ii) Lösen oder Dispergieren von 2 - 10 % (Gew./Vol.) des lipophilen Stoffs oder lipophilen Stoffgemischs im Lösungsmittel und Homogenisieren:
2 - 10 % (Gew./Vol.) des lipophilen Stoffs oder lipophilen Stoffgemischs (z.B. Wirkstoffs oder Pflanzenextrakts) werden im Lösungsmittel, bevorzugt Ethanol gelöst oder dispergiert. Die Mischung wird bevorzugt bei 5.000 U/min für 5 Minuten homogenisiert, um eine gleichmäßige Verteilung sicherzustellen. Die Temperatur sollte dabei maximal 30°C sein, um temperaturempfindliche Komponenten zu schützen. Bevorzugt ist ein Temperaturbereich von 5°C bis 15°C. Anschließend wird die Mischung mit einem Energieeintrag von beispielsweise 10.000 W netto pro 100 mL Lösung mit Ultraschall beschallt. Die Temperatur sollte dabei maximal 30°C sein, um temperaturempfindliche Komponenten zu schützen. Bevorzugt ist ein Temperaturbereich von 5°C bis 15°C.
iii) Zufügen von 20 - 50 % (Gew./Vol.) des Gummi arabicums;
iv) Zufügen von 1 - 5 % (Gew./Vol.) des Chitosans;
v) Zufügen von 2 - 10 % (Gew./Vol.) des Aloe vera-Gels/Pulvers;
vi) Homogenisieren,
   Bevorzugt wird 20 - 50% (Gew./Vol.) sprühgetrocknetes GA-Pulver hinzugegeben. Die Mischung kann optional für bevorzugt 1 Minute bei beispielsweise 10.000 U/min homogenisiert werden, wobei dies nicht zwingend notwendig ist. Die Temperatur sollte dabei maximal 30°C sein. Bevorzugt ist ein Temperaturbereich von 5°C bis 15°C. Anschließend wird 1 - 5 % (Gew./Vol.) des Chitosans hinzugefügt. Die Mischung kann optional für bevorzugt 1 Minute bei beispielsweise 10.000 U/min homogenisiert werden, wobei dies nicht zwingend notwendig ist. Die Temperatur sollte dabei maximal 30°C sein. Bevorzugt ist ein Temperaturbereich von 5°C bis 15°C. Daraufhin erfolgt die Zugabe von 2 - 10 % (Gew./Vol.) des Aloe vera-Gels/Pulvers. Die Mischung wird bevorzugt für 5 Minuten bei beispielsweise 10.000 U/min homogenisiert. Die Temperatur sollte dabei maximal 30°C sein. Bevorzugt ist ein Temperaturbereich von 5°C bis 15°C.
vii) optionales Entgasen;
   Das entgasen in Schritt vii) kann mittels Ultraschallbehandlung erfolgen. Hierfür wird die Mischung mit einem Energieeintrag von bevorzugt 10.000 W netto pro 100 mL Lösung mittels Ultraschalls behandelt. Dieser Schritt dient dazu, eine gleichmäßigere Durchmischung von GA, Chitosan und AV und Wirkstoff zu erreichen und um eventuell vorhandene Aggregate aufzubrechen. Die Temperatur sollte dabei maximal 30°C sein. Bevorzugt ist ein Temperaturbereich von 5°C bis 15°C.
viii) Entfernen des Lösungsmittels,
   In Schritt viii) erfolgt abschließend die Entfernung des Lösungsmittels z.B. durch Destillation. Bevorzugt wird als Lösungsmittel Ethanol, insbesondere lebensmitteltaugliches Ethanol verwendet. Die Abdestillation des Ethanols kann wie folgt erfolgen. Das Ethanol wird im Rotationsverdampfer bei 50 mbar, 200 U/min und 30°C entfernt. Durch den Verdampfungsprozess wird das Ethanol zurückgewonnen, während das GA-Chitosan-AV-Wirkstoff-Pulver als feines, homogenes Feststoffgemisch (Pulver) zurück bleibt.

Die vorliegende Erfindung betrifft zudem eine Mikroemulsion erhalten oder erhältlich nach einem der hierin beschriebenen erfindungsgemäßen.

### Beispiele

### Beispiel 1: Herstellung der Trägerlösung

### 1.1 Benötigte Rohstoffe zur Herstellung der Trägerlösung

### Wasser:

Zur Entfernung von Verunreinigungen wird das Wasser durch Umkehrosmose und Filtration aufbereitet. Dieser Schritt gewährleistet eine hohe Reinheit des Wassers und minimiert potenzielle Störeinflüsse auf die Emulsionsqualität.

### Calciumchlorid (CaCl₂):

Calciumchlorid in Lebensmittelqualität wird zur Herstellung der Trägerlösung eingesetzt. Calciumionen (Ca²⁺) sind vorteilhaft zum Steigern der Ionenstärke der Lösung, wodurch elektrostatische Wechselwirkungen innerhalb des Systems gezielt beeinflusst werden. Dies verbessert insbesondere die Bindung zwischen anionischen Gruppen (z. B. Carboxylgruppen von Polysacchariden wie Acemannan aus Aloe vera oder AGPs aus Gummi arabicum) und kationischen Molekülen (z. B. Chitosan). Eine erhöhte Ionenstärke kann die Vernetzung und Stabilisierung der Emulsion fördern, indem sie die elektrostatischen Bindungen an den Grenzflächen stärkt. Dies verbessert die mechanische Festigkeit der Grenzflächenschicht und erhöht die physikalische Integrität der Tropfenstruktur. Zu hohe Ionenstärken können jedoch destabilisieren, indem sie die elektrostatische Abschirmung zu stark erhöhen.

### Lecithin:

Hochreines, fettfreies, degummiertes (entschleimtes) Lecithin pflanzlichen Ursprungs aus Sonnenblumen (Sonnenblumenlecithin) oder Sojabohnen (Sojalecithin) wurde zur Herstellung der Trägerlösung eingesetzt. Zur Herstellung der Trägerlösung wurden Lecithin mit einem Phosphatidylcholingehalt von mindestens 30 % eingesetzt. Diese Eigenschaft hat sich als entscheidend erwiesen, da Phosphatidylcholin ein wesentlicher Emulgator ist, der die Mizellisierung von lipophilen Pflanzenextrakten fördern kann. In den vorliegenden Beispielen wurde Lecithin verwendet, das einen Phosphatidylcholingehalt von mindestens 45 Gew.-% aufweist, da sich ein Gehalt an Phosphatidylcholin von 45% Gew.-% als besonders vorteilhaft erwiesen hat.

### 1.2 Herstellung der Trägerlösung

Das Wasser (z.B. Reinstwasser) wird auf 35°C erwärmt. Das Erwärmen des Wassers auf 35°C sorgt für eine bessere Löslichkeit, eine beschleunigte Hydratation und eine effiziente Homogenisierung.
2 - 3% (Gew./Vol.) Lecithin mit einem Phosphatidylcholingehalt von mindestens 30 % wird dem Wasser schrittweise zugesetzt und unter kontinuierlichem Rühren bei (z.B. 1.000 U/min) vollständig gelöst.
0,1 bis 0,3% (Gew./Vol.) an physiologisch verträglichem Salz (z.B. CaCl₂), entsprechend einer Konzentration von ca. 9 bis 27 mM, wird der Lösung zugesetzt und unter Rühren vollständig aufgelöst.

### 1.3 Entgasung der Trägerlösung

Nach der Herstellung der Trägerlösung gemäß dem allgemeinen Protokoll ist eine möglichst vollständige Entgasung erforderlich, um gelöste Gase zu entfernen, die die Selbstassemblierung der Hydrokolloide beeinträchtigen könnten.

Zu diesem Zweck kann die Trägerlösung in eine Unterdruckkammer überführt und dort einem Unterdruck von weniger als 50 mbar ausgesetzt. Während der Entgasung kann die Lösung kontinuierlich gerührt werden, um eine gleichmäßige Freisetzung der Gase aus dem gesamten Volumen der Lösung zu gewährleisten. Dieser Prozess wird bevorzugt für eine Dauer von ca. 15 Minuten durchgeführt, wobei die genaue Zeitdauer je nach Gesamtvolumen der Lösung angepasst werden muss.

Alternativ kann die Lösung mittels Ultraschalls im Eisbad entgast werden, wobei darauf zu achten ist, dass die Ultraschallintensität nicht zu hoch gewählt wird. Das Eisbad dient dazu, die Temperatur während des Prozesses niedrig zu halten und hitzelabile Komponenten zu schützen. Ein zu hoher Energieeintrag kann empfindliche Komponenten der Trägerlösung beschädigen, abbauen oder die elektrostatischen Bindungen zwischen ihnen beeinträchtigen.

### Beispiel 2: Herstellung des Pulverkonzentrats

### 1 Benötigte Rohstoffe zur Herstellung des Pulverkonzentrats

### Gummi arabicum:

Es wurde sprühgetrocknetes Gummi arabicum vom Typ *Acacia senegal* mit einem Gehalt an AGPs von mindestens 3% eingesetzt.

### Chitosan:

Es wurde Chitosan fungalen Ursprungs eingesetzt, das aus den Zellwänden der Pilzarten *Mucor spp.* oder *Rhizopus spp.* gewonnen wurde. Das eingesetzte Chitosan weist einen Grad der Deacetylierung von > 85 % auf, der die kationischen Eigenschaften und die Wechselwirkung mit den anionischen AGPs aus GA verbessert.

### Aloe vera:

Es wurde sprühgetrocknetes Aloe-vera-Pulver aus dem Inneren der Blätter von Aloe barbadensis Miller verwendet. Das Pulver weist einen Gehalt an bioaktiven Polysacchariden, insbesondere Acemannan (> 15 %), auf, da diese die physikalische Stabilität der Emulsion fördern können.

### Ethanol:

Es wurde lebensmitteltaugliches Ethanol verwendet.

### Lipophiler Stoff/Lipophiles Stoffgemisch:

Folgende lipophile Stoffe bzw. lipophile Stoffgemische wurden bei der Herstellung verschiedener Pulverkonzentrate verwendet: Hanfextrakte, Cannabinoide (Isolate), Curcuma-Extrakte, Curcuminoide (Isolate), Weihrauch-Extrakte, Boswelliasäuren (Isolate), Coenzym Q10, Ingwer-Extrakte, Ashwagandha-Extrakte, Oligomere Proanthocyanidine, Resveratrol, Vitamin A, Vitamin D, Vitamin E, Vitamin K, omega-3 Fettsäuren aus Fischöl oder Algenöl, Schwarzkümmelöl, Schwarzkümmelextrakte, Artemisia-Extrakte, Extrakte oder Ätherische Öle aus Oregano, Curcuma, Weihrauch, Ingwer, Lavendel, Thymian, Zitrone, Orange, Pfefferminze, Rosmarin, Eukalyptus, Basilikum, Schwarzkümmel, Nelke, Ylang-Ylang, Anis, Kardamon, Salbei, Zitronengras, Wachholderbeeren, Bergamotte, Zitronenverbene, Johanniskraut, Astaxanthin, Spermidin, Quercetin, Fisetin und NAD+.

### 1.2 Herstellung des Pulverkonzentrates

Die genauen Mengen an GA, Chitosan und Aloe Vera wurden wirkstoffspezifisch in Reihenversuchen ermittelt, um eine optimale Stabilisierung zu erreichen. Zu wenig GA führt dazu, dass die Wirkstoffe ausfallen, wenn das GA-Wirkstoff-Pulver im nachfolgenden Schritt in Wasser gelöst wird. Dies ist darauf zurückzuführen, dass die AGPs aus GA an der Grenzfläche eine stabilisierende Schutzschicht um hydrophobe Wirkstoffpartikel bilden. Fehlt es an einer ausreichenden Menge an GA, bleibt die Schutzschicht unvollständig, wodurch hydrophobe Wirkstoffe unkontrolliert aggregieren und schließlich ausfallen können. Eine übermäßige Zugabe von GA hingegen erhöht die Viskosität der Lösung signifikant, was das Self-Assembling der Hydrokolloide behindert. Dieses Self-Assembling, ein zentraler Prozess bei der Bildung von Mizellen, erfordert ein möglichst niedervisköses Medium, das ausreichend Bewegungsfreiheit für die Moleküle bietet. Eine hochvisköse Lösung schränkt diese Dynamik ein und führt zu einer ineffizienten Bildung stabiler Mizellenstrukturen.

Lösen oder Dispergieren des Wirkstoffs oder Extrakts:
2 - 10% (Gew./Vol.) des Wirkstoffs oder Pflanzenextrakts werden in Ethanol gelöst oder dispergiert. Die Mischung wird bei 5.000 U/min für 5 Minuten homogenisiert, um eine gleichmäßige Verteilung sicherzustellen. (TMAX: 30°C, bevorzugt 5°C bis maximal 15°C) Die Mischung wird mit einem Energieeintrag von 10.000 W netto pro 100 mL Lösung mit Ultraschall beschallt. (TMAX: 30°C, bevorzugt 5°C bis maximal 15°C)

Zugabe von Gummi arabicum, Chitosan und Aloe Vera:
20 - 50% (Gew./Vol.) sprühgetrocknetes GA-Pulver werden hinzugegeben. Die Mischung wird für 1 Minute bei 10.000 U/min homogenisiert. (nicht zwingend notwendig) / (TMAX: 30°C, bevorzugt 5°C bis maximal 15°C)
1 - 5% (Gew./Vol.) Chitosan werden hinzugefügt. Die Mischung wird für 1 Minute bei 10.000 U/min homogenisiert. (nicht zwingend notwendig) / (TMAX: 30°C, bevorzugt 5°C bis maximal 15°C)
2 - 10% Aloe Vera-Gel/Pulver werden hinzugefügt. Die Mischung wird für 5 Minuten bei 10.000 U/min homogenisiert. (TMAX: 30°C, bevorzugt 5°C bis maximal 15°C)

Ultraschallbehandlung:
Die Mischung wird mit einem Energieeintrag von 10.000 W netto pro 100 mL Lösung mittels Ultraschalls behandelt. Dieser Schritt dient dazu, eine gleichmäßigere Durchmischung von GA, Chitosan und AV und Wirkstoff zu erreichen und um eventuell vorhandene Aggregate aufzubrechen. (TMAX: 30°C, bevorzugt 5°C bis maximal 15°C)

Abdestillation des Ethanols:
Das Ethanol wird im Rotationsverdampfer bei 50 mbar, 200 U/min und 30°C entfernt. Durch den Verdampfungsprozess wird das Ethanol zurückgewonnen, während das GA-Chitosan-AV-Wirkstoff-Pulver als feines, homogenes Feststoffgemisch (Pulver) zurück bleibt

### Beispiel 3: Herstellung einer stabilen Mikroemulsion

Sämtliche Schritte wurden in einem Eisbad durchgeführt, um die Stabilität von temperaturempfindlichen Komponenten sicherzustellen. Im folgenden Schritt wird das Pulverkonzentrat aus Beispiel 2 in die Trägerlösung aus Beispiel 1 eingearbeitet.

Ein Maximum von 30% (Gew./Vol.) des Pulverkonzentrates wird in der Trägerlösung schrittweise und unter kontinuierlichem Rühren bei 10.000 U/min gelöst. Die hierbei eingesetzte Menge an Pulverkonzentrat hängt von der gewünschten Konzentration des Wirkstoffes/Extraktes im fertigen Solubilisat ab. (TMAX: 30°C, bevorzugt 5°C bis maximal 15°C) Nach der vollständigen Zugabe wird die Mischung für weitere 15 Minuten bei 10.000 U/min homogenisiert. (TMAX: 30°C, bevorzugt 5°C bis maximal 15°C). pH wird auf einen Wert von 4.0 eingestellt. Je nach Wirkstoff oder Extrakt ergibt dieser Schritt Hydrokolloide mit einem durchschnittlichen hydrodynamischen Durchmesser zwischen 800 nm und 1.200 nm.

Optional: Um eine kleinere Partikelgröße im Bereich zwischen 200 nm und 800 nm zu erreichen, kann anschließend eine Ultraschallbehandlung eingesetzt werden. Die optimale Ultraschallintensität und der erforderliche Energieeintrag müssen jedoch in Vorversuchen bestimmt werden. Dazu wird nach verschiedenen Energieeinträgen die Partikelgröße mittels dynamischer Lichtstreuung (DLS) analysiert. Es ist wichtig zu beachten, dass eine zu hohe Ultraschallintensität die Hydrokolloidstrukturen zerstören kann.

Zusätzlich kann eine Sedimentationskontrolle durch Zentrifugation durchgeführt werden, um die physikalische Stabilität der Hydrokolloide zu überprüfen. Dazu werden z.B. 10 mL der Lösung für 60 Sekunden bei 2.500 × g zentrifugiert. Es sollte sich dabei kein Pellet bilden; ein Ausfallen der Partikel würde auf eine unzureichende Stabilisierung hinweisen.

Die Mikroemulsion enthält 1 - 5% (Gew./Vol.) Wirkstoff/Extrakt, 10 - 25% (Gew./Vol.) Gummi arabicum, 0,5 - 1% (Gew./Vol.) Chitosan, 1 - 5% (Gew./Vol.) Aloe Vera-Gel/Pulver, 2 - 3% (Gew./Vol.) Lecithin und 0,1 - 0,3% (Gew./Vol.) CaCl₂.

### Beispiele für geeignete Geräte

Homogenisator: Silverson L5M-A
Ultraschall: Hielscher UP400St
Rotationsverdampfer (Rotovapor): IKA RV 8 Complete
Dynamic Light Scattering (DLS): Anton Paar Litesizer 500 oder 700

### Beispiel 4: Herstellung eines 2%igen Curcumin-Solubilisates (Endvolumen: 1.000 mL)

### 4.1 Herstellung der Trägerlösung

1.000 mL Reinstwasser wird auf 35°C erwärmt.

20 g Lecithin wird schrittweise unter Rühren bei 1.000 U/min (Homogenisator oder Magnetrührer) gelöst.

0,1 bis 0,3% (Gew./Vol.) an CaCl₂, entsprechend einer Konzentration von ca. 9 bis 27 mM, wird der Lösung zugesetzt und unter Rühren vollständig aufgelöst.
Die Lösung wird mittels Ultraschalls entgast (z.B. 22mm Sonotrode, Amplitude: 100%, 15 Minuten, ca. 50 Watt)

### 4.2 Herstellung des Pulverkonzentrates

20 g Curcumin werden in 500 mL Ethanol gelöst/dispergiert.

Homogenisierung bei 5.000 U/min für 5 Minuten.

Ultraschallbehandlung: 100.000 W netto.

240 g Gummi arabicum werden der Mischung hinzugefügt.

Homogenisierung bei 10.000 U/min für 1 Minute.

20 g Chitosan werden der Mischung hinzugefügt.

Homogenisierung bei 10.000 U/min für 1 Minute.

20 g Aloe Vera-Pulver werden der Mischung hinzugefügt.

Homogenisierung bei 10.000 U/min für 5 Minuten.

Abdestillation des Ethanols: Das Ethanol wird im Rotationsverdampfer bei 50 mbar, 200 U/min und 30°C entfernt.

### 7.4 Herstellung der Mikroemulsion

300 g des Pulverkonzentrates werden in 600 mL Trägerlösung schrittweise unter kontinuierlichem Rühren bei 10.000 U/min gelöst.

Nach der vollständigen Zugabe wird die Mischung für weitere 15 Minuten bei 10.000 U/min homogenisiert.

Volumen wird auf 1.000 mL justiert und nochmals kurz homogenisiert.

pH wird auf 4.0 eingestellt
DLS-Messung zur Kontrolle der Partikelgrössenverteilung (hydrodynamischer Durchmesser sollte bei 800 - 1.200 nm liegen)
Optionale Ultraschallbehandlung zur Reduktion der Partikelgrösse:
   Beispiel: 50 mL des Solubilisates werden mit einer 7mm Sonotrode bei einer Amplitude von 100% für 30 Minuten beschallt (hydrodynamischer Durchmesser sollte nach der Behandlung zwischen 200 und 800 nm liegen)

## Patentansprüche

1. Verfahren zur Herstellung einer Mikroemulsion eines lipophilen Stoffes oder lipophilen Stoffgemisches umfassend die Schritte:
A) Bereitstellen einer Trägerlösung umfassend oder bestehend aus einer wässrigen Lösung eines physiologisch verträglichen Elektrolytsalzes und Lecithin mit einem Phosphatidylcholingehalt von mindestens 30 %;
B) Bereitstellen eines Pulverkonzentrats umfassend oder bestehend aus Gummi arabicum mit einem Gehalt an Arabinogalactanprotein von mindestens 3 Gew.-%, Chitosan mit einem Deacetylierungsgrad von mindestens 70 %, Aloe vera-Gel/Pulver mit einem Gehalt an Acemannan von mindestens 10 Gew.-% bezogen auf die Trockenmasse; und dem lipophilen Stoff oder dem lipophilen Stoffgemisch;
C) Zufügen von maximal 30% Gew./Vol. des Pulverkonzentrats zur Trägerlösung zum Herstellen einer Pulverkonzentrat-Trägerlösung;
D) Homogenisieren der Pulverkonzentrat-Trägerlösung unter Bildung einer Mikroemulsion, und wobei ein Einstellen des pH-Werts auf 4,0 erfolgt.

2. Das Verfahren gemäß Anspruch 1, wobei das Verfahren weiter den Schritt E) umfasst:
E) Behandeln der Mikroemulsion mit Ultraschall unter Bildung einer Mikroemulsion enthaltend Hydrokolloide mit einem durchschnittlichen hydrodynamischen Durchmesser zwischen 200 nm und 800 nm.

3. Das Verfahren gemäß Anspruch 1 oder 2, wobei die Trägerlösung 2 - 3 % Gew./Vol. Lecithin enthält.

4. Das Verfahren gemäß einem der Ansprüche 1 - 3, wobei das physiologisch verträgliche Elektrolyt-Salzes ausgewählt wird aus der Gruppe umfassend oder bestehend aus Calciumchlorid, Natriumchlorid, Magnesiumchlorid, und Kaliumchlorid, bevorzugt Calciumchlorid.

5. Das Verfahren gemäß einem der Ansprüche 1 - 4, wobei die Trägerlösung das physiologisch verträgliche Elektrolyt-Salz in einer Konzentration von ca. 9 bis 27 mM enthält.

6. Das Verfahren gemäß einem der Ansprüche 1 - 5, wobei das Chitosan einen Deacetylierungsgrad mindestens 85% aufweist.

7. Das Verfahren gemäß einem der Ansprüche 1 - 6, wobei Aloe vera-Gel/Pulver einen Gehalt an Acemannan von mindestens 15 Gew.-% aufweist.

8. Das Verfahren gemäß einem der Ansprüche 1 - 7, wobei das Lecithin einen Phosphatidylcholingehalt von mindestens 45 Gew.-% aufweist.

9. Das Verfahren gemäß einem der Ansprüche 1 - 8, wobei die Trägerlösung hergestellt wird durch ein Verfahren umfassend die Schritte:
a) Bereitstellen von Wasser, eines physiologisch verträglichen Elektrolytsalzes, bevorzugt Calciumchlorid, und Lecithin mit einem Phosphatidylcholingehalt von mindestens 30 %;
b) Temperieren des Wassers auf eine Temperatur zwischen 30°C - 45°C, bevorzugt 35°C;
c) Zufügen von 2 - 3 % Gew./Vol. des Lecithins;
d) Zufügen von 0,1 - 0,3 % Gew./Vol. des physiologisch verträglichen Elektrolytsalzes, bevorzugt Calciumchlorid;
e) Entgasen der Trägerlösung.

10. Das Verfahren gemäß einem der Ansprüche 1 - 9, wobei das Pulverkonzentrat hergestellt wird durch ein Verfahren umfassend die Schritte:
i) Bereitstellen von Lösungsmittel, Gummi arabicum mit einem Gehalt an Arabinogalactanprotein von mindestens 3 Gew.-%, Chitosan mit einem Deacetylierungsgrad von mindestens 70%, Aloe vera-Gel/Pulver mit einem Gehalt an Acemannan von mindestens 10 Gew.-% bezogen auf die Trockenmasse; und lipophilen Stoff oder lipophilen Stoffgemisch;
ii) Lösen oder Dispergieren von 2 - 10 % Gew./Vol. des lipophilen Stoffs oder lipophilen Stoffgemischs im Lösungsmittel und Homogeniseren;
iii) Zufügen von 20 - 50 % Gew./Vol. des Gummi arabicums;
iv) Zufügen von 1 - 5 % Gew./Vol. des Chitosans;
v) Zufügen von 2 - 10 % Gew./Vol. des Aloe vera-Gels/Pulvers;
vi) Homogenisieren;
vii) optionales Entgasen; und
viii) Entfernen des Lösungsmittels.

11. Das Verfahren gemäß Anspruch 10, wobei das Lösungsmittel Ethanol ist.

12. Das Verfahren gemäß einem der Ansprüche 1 - 9, wobei Schritt C) bei einer Temperatur von ≤ 15°C durchgeführt wird.

13. Das Verfahren gemäß einem der Ansprüche 1 - 12, wobei die Mikroemulsion 1 - 5% Gew./Vol. lipophilen Stoff oder lipophiles Stoffgemisch, 10 - 25% Gew./Vol. Gummi arabicum, 0,5 - 1% Gew./Vol. Chitosan, 1 - 5% Gew./Vol. Aloe Vera-Gel/Pulver, 2 - 3% Gew./Vol. Lecithin und 0,1 - 0,3% Gew./Vol. Calciumchlorid enthält.

14. Das Verfahren gemäß einem der Ansprüche 1 - 13, wobei der lipophile Stoff oder das lipophile Stoffgemisch ausgewählt werden aus der Gruppe umfassend oder besteht aus Hanfextrakte, Cannabinoide, Curcuma-Extrakte, Curcuminoide, Weihrauch-Extrakte, Boswelliasäuren, Coenzym Q10, Ingwer-Extrakte, Ashwagandha-Extrakte, Oligomere Proanthocyanidine, Resveratrol, Vitamin A, Vitamin D, Vitamin E, Vitamin K, omega-3 Fettsäuren aus Fischöl oder Algenöl, Schwarzkümmelöl, Schwarzkümmelextrakte, Artemisia-Extrakte, Extrakte oder Ätherische Öle aus Oregano, Curcuma, Weihrauch, Ingwer, Lavendel, Thymian, Zitrone, Orange, Pfefferminze, Rosmarin, Eukalyptus, Basilikum, Schwarzkümmel, Nelke, Ylang-Ylang, Anis, Kardamon, Salbei, Zitronengras, Wachholderbeeren, Bergamotte, Zitronenverbene, Johanniskraut, Astaxanthin, Spermidin, Quercetin, Fisetin und NAD+.

15. Mikroemulsion erhalten oder erhältlich nach einem Verfahren gemäß einem der Ansprüche 1 - 14.
